# EUROPEAN PATENT SPECIFICATION

(11) **EP 1 178 822 B1**
(45) Date of publication and mention of the grant of the patent: **18.02.2004**
(21) Application number: 00931400.6
(22) Date of filing: 19.05.2000
(51) Int. Cl.: A61K 39/00, A61P 35/00, C12N 5/00

(54) **WHOLE CELL CANCER VACCINE COMPRISING CO-CULTURED MALIGNANT AND NON-MALIGNANT CELLS**
AUS GANZEN ZELLEN BESTEHENDER KREBS-IMPFSTOFF, ENTHALTEND KO-KULTIVIERTE MALIGNE UND NICHT-MALIGNE ZELLEN
VACCIN ANTICANCEREUX A CELLULES ENTIERES COMPRENANT DES CELLULES MALIGNES ET NON-MALIGNES CO-CULTIVEES

(30) Priority: 21.05.1999 GB 9911823; 21.05.1999 GB 9911824; 01.04.2000 GB 0008029; 01.04.2000 GB 0008032
(43) Date of publication of application: 13.02.2002
(73) Proprietor: Onyvax Limited, London SW17 0RE (GB)
(72) Inventor: SMITH, Peter, Onyvax Limited, London SW17 0RE (GB); STEVENSON, Darren, Onyvax Limited, London SW17 0RE (GB); CHANA, Haj, Onyvax Limited, London SW17 0RE (GB); THRAVES, Peter, Onyvax Limited, London SW17 0RE (GB); SUTTON, Andrew, London, SW17 0RE (GB)
(74) Representative: Davies, Jonathan Mark
(86) International application number: PCT/GB2000/001918
(87) International publication number: WO 2000/071155

(56) References cited:
- WO-A-97/24132
- WO-A-97/28255
- WO-A-99/19462
- US-A- 5 804 443
- OLUMI ARIA F ET AL: "A novel coculture technique demonstrates that normal human prostatic fibroblasts contribute to tumor formation of LNCaP cells by retarding cell death." CANCER RESEARCH, vol. 58, no. 20, 15 October 1998 (1998-10-15), pages 4525-4530, XP002152739 ISSN: 0008-5472
- FRESHNEY R. I.: "Culture of animal cells" 1987 , ALAN R. LISS , US, NEW YORK XP002152704 154920 page 295, left-hand column, line 26 -right-hand column, line 2 page 314, left-hand column, line 3 - line 17

## Description

### Summary

New vaccines for use as immunotherapeutic agents in the treatment of cancer are described. First generation whole cell vaccines relied on traditional methodologies in the culture and production of the autologous or allogeneic cell based vaccines, whereby autologous tumour cells or immortalised allogeneic tumour cell lines were cultured in static two dimensional (2D) culture conditions. This invention relates to a co-culture techniques which leads to significant enhancement of the phenotype of cell lines towards a more *in vivo* phenotype resulting in a vaccine reflecting a more realistic phenotypic representation of an *in vivo* tumour. We describe alternative culture techniques which offer significant scale up and processing benefits and in addition significantly improve the activity of the whole-cell vaccine. Furthermore vaccines produced by the various co-culture techniques described have additional advantages over single cell suspension vaccines in the mode of presentation to the immune system which leads to enhanced performance in treating the disease.

### Background to the Invention

It is known in the art that cancerous cells contain numerous mutations, qualitative and quantitative, spatial and temporal, relative to their non-malignant, non-cancerous counterparts and that at certain periods during tumour growth and spread a proportion of the cancerous cells are capable of being recognised by the host's immune system as abnormal. Cancer cells express certain tumour-specific antigens (TSA) and tumour-associated antigens (TAA) that may serve as markers or targets for the immune system. This has led to numerous research efforts worldwide to develop immunotherapies that harness the power of the immune system and direct it to attack the cancerous cells, with the aim of eliminating such aberrant cells at least to a level that is not life-threatening (reviewed in Maraveyas, A. & Dalgleish, A.G. 1977 Active immunotherapy for solid tumours in vaccine design in The Role of Cytokine Networks, Ed. Gregoriadis et al., Plenum Press, New York, pages 129-145; Morton, D.L. and Ravindranath, M.H. 1996 Current concepts concerning melanoma vaccines in Tumor Immunology - Immunotherapy and Cancer Vaccines, ed. Dalgleish, A.G. and Browning, M., Cambridge University Press, pages 241-268. See also other papers in these publications for further detail).

Tumours have the remarkable ability to counteract the immune system in a variety of ways which may include: down-regulation of the expression of potential target proteins; mutation of potential target proteins; down-regulation of surface expression of receptors and other proteins; down-regulation of MHC class I and II expression thereby disallowing direct presentation of TAA or TSA peptides; down-regulation of co-stimulatory molecules leading to incomplete stimulation of T-cells leading to anergy; shedding of selective, non representative membrane portions to act as decoy to the immune system; shedding of selective membrane portions to anergise the immune system; secretion of inhibitory molecules; induction of T-cell death; and many other ways. What is clear is that the phenotypic and genetic heterogeneity and plasticity of tumours in the body will have to be matched to a degree by immunotherapeutic strategies which similarly embody heterogeneity or polyvalency. The use of whole cancer cells, or crude derivatives thereof, as cancer immunotherapies can be viewed as analogous to the use of whole inactivated or attenuated viruses as vaccines against viral disease. The potential advantages are:
(a) whole cells contain a broad range of antigens, providing an antigenic profile of sufficient heterogeneity to match that of the lesions as described above;
(b) being multivalent (i.e. containing multiple antigens), the risk of immunological escape is reduced (the probability of cancer cells 'losing' through mutation all of these antigens is remote); and
(c) cell-based vaccines include TSAs and TAAs that have yet to be identified as such; it is possible if not likely that currently unidentified antigens may be clinically more relevant than the relatively small number of TSAs/TAAs that are known.

Cell-based vaccines fall into two categories. The first, based on autologous cells, involves the removal of a biopsy from a patient, cultivating tumour cells *in vitro*, modifying the cells through transfection and/or other means, irradiating the cells to render them replication-incompetent and then injecting the cells back into the same patient as a vaccine. Although this approach enjoyed considerable attention over the past decade, it has been increasingly apparent that this individually-tailored therapy is inherently impractical for several reasons. The approach is time consuming (often the lead-time for producing clinical doses of vaccine exceeds the patients life expectancy), expensive and, as a 'bespoke' product, it is not possible to specify a standardised product (only the procedure, not the product, can be standardised and hence optimised and quality controlled). Furthermore, the tumour biopsy used to prepare the autologous vaccine will have certain growth characteristics, interactions and communication with surrounding tissue that makes it somewhat unique. This alludes to a potentially significant disadvantage to the use of autologous cells for immunotherapy: a biopsy which provides the initial cells represents an immunological snapshot of the tumour, in that environment, at that point in time, and this may be inadequate as a phenotypic representation over time for the purpose of a vaccine with sustained activity that can be given over the entire course of the disease.

The second type of cell-based vaccine describes the use of allogeneic cells which are be genetically (and hence immunologically) mismatched to the patients. Allogeneic cells benefit from the same advantages of multivalency as autologous cells. In addition, as allogeneic cell vaccines can be based on immortalised cell lines which can be cultivated indefinitely *in vitro,* this approach does not suffer the lead-time and cost disadvantages of autologous approaches. Similarly the allogeneic approach offers the opportunity to use combinations of cells types which may match or anticipate the disease profile of an individual in terms of stage of the disease, the location of the lesion and potential resistance to other therapies.

Previous human clinical studies utilising whole cell based approaches have relied upon growth and production of the cells in traditional 2D culture systems - cells grown in static plastic flasks within incubators, with or without CO₂ atmospheric supplementation (termed "2D" culture herein). The cells grow in two dimensions only as a flat monolayer or sheet of cells adherent to the plastic or glass vessel. Cell growth usually becomes contact inhibited at confluence and attempts to grow the cells beyond this point usually results in the loss of the culture. The growth of cells in 2D culture relies on the serial passage of cells, wherein the cells are repeatedly removed from the substratum and subjected to a harsh cycle of centrifugation, washing and manipulation in suspension. The cells are grown and then harvested by mechanical means or treatment with trypsin to remove the cells from the substratum. The effect of trypsin, other proteolytic enzymes or other agents used for the purpose is to break attachment of the cells to the flask substrate. Proteolytic treatment inevitably removes some protein molecules from the surface of the cells, which may result in loss of potential epitopes that enable the recipient to raise an humoral or cell based immune response to the tumour. For example, proteolysis may remove MHC-I molecules that could present peptides in a direct manner to patient T cells, or adhesion molecules that offer co-stimulatory activity to direct presentation. Alternatively proteolysis may simply remove aberrantly expressed TAA or TSA which when processed through a cross-priming or other route could also contribute to an anti-tumour response. Furthermore, there is relatively little opportunity to manipulate the cell phenotype since, in order to attain the quantity of cells required, a number of passages under standard growth conditions are required and attempts to drive the cells into a stressed or apoptotic or necrotic or otherwise altered phenotype prior to harvest results in significant losses of fragile cells at harvest undermining the feasibility of producing vaccine at a practical scale.

Hitherto described cell based cancer vaccines are based on i) populations of primary cultures of autologous tumour cells; ii) single populations of allogeneic immortalised tumour cell lines; iii) allogeneic immortalised cell lines as many as three or four in combination and iv) genetically modified fibroblasts in combination with tumour cells.

An example of previous approaches is provided by WO/99 19462. This application discloses a whole cell cancer vaccine wherein the malignant cells are cultured with other non-malignant cells. This results in a vaccine in which the non-malignant cells have an enhanced cytokine secretion and co-stimulatory molecules expression, which renders the whole cell vaccine more effective.

The present invention describes culture methods for large scale production of vaccines; phenotype adjustment of established cell lines; co-culture techniques; specific compositions of immortalised tumour cell lines and immortalised non-malignant cells; compositions comprising immortalised tumour cells and autologous non-malignant cells; processing methods for the production of the vaccines; analytical techniques to evaluate phenotype adjustment and new compositions which display increased immunogenicity in standard models of tumour prevention and therapy. In addition, this invention describes a new strategy, targeting the immune response not only to the tumour itself but also to the local tumour environment and the non-malignant cells which comprise that environment.

### Description of the Invention

It is well known that the immediate local tumour environment is intensely immunosuppressive where all facets of immune surveillance are inhibited, from inhibition of T cell and antigen presenting cell (APC) migration, to inhibition of APC maturation and induction of T cell anergy. Many of the immunosuppressive activities found in the immediate local tumour environment are mediated by soluble factors released by the tumour cells, such as IL-10 and TGFβ. There is abundant literature which reports the effects of tumour cells on adjacent non-malignant cells, whether they be epithelia, stromal fibroblasts, endothelial cells or any other cell type as relevant. Tumour-adjacent cells are induced to secrete or express enhanced levels of receptors or other proteins (e.g. Tumour and stromal expression of matrix metalloproteinases and their role in tumour progression reviewed by H.C. Crawford in Invasion Metastasis, (1994), 14, p234-245; Expression of uPA and its receptor by both neoplastic and stromal cells during xenograft invasion, Int. J. Cancer. (1994), 57, p553-560; Vitronectin in colorectal adenocarcinoma, synthesis by stromal cells in culture, Experimental Cell Research (1994), 214, p303-312; Invasive tumours induce c-ets1 transcription factor expression in adjacent stroma, Biol. Cell. (1995), 84, p53-61; Tumour cell and connective tissue cell interactions in human colorectal adenocarcinoma, Am. J. Pathology (1997), 151, p479-493; CD40 antigen is expressed in endothelial cells and tumour cells in Kaposi's sarcoma, Am. J. Pathology (1996), 148, p1387-1397; Stromelysin is overexpressed by stromal elements in primary non-small cell lung cancers and is regulated by retinoic acid in pulmonary fibroblasts, Cancer Research, (1995), 55, p4120-4126; Increased expression of epidermal growth factor receptor in non-malignant epithelium adjacent to head and neck carcinomas, Oral Diseases, (1998),4,p4-8). The tumour-adjacent non-malignant cells are aberrant to distant non-malignant cells in that they are induced to express proteins that distant counterparts do not; this will be reflected in a qualitatively and/or quantitatively modified spectrum of peptides displayed on the tumour-adjacent cells MHC-I molecules which can be recognised by the immune system as aberrant. Thus, in a similar way to anti-angiogenesis based strategies, this embodiment targets the local tumour micro-environment in addition to the tumour itself.

This embodiment describes the preparation of a mixed cell type vaccine, in which tumour cell lines are grown in intimate contact with a non-malignant component. The growth of this mixed cell type vaccine can be achieved by a number of means, for example co-culture in 2D flasks, growth in transwell inserts where conditioned medium exerts its effect on the tumour line or growth in a 3D mixed culture system. There are significant benefits to a mixed culture approach in that the literature reports that in many cases celi-ceil contact is required to elicit reciprocal phenotypic changes in both tumour and non-tumour components (e.g. Fibroblast mediated differentiation in human breast carcinoma cells (MCF-7) grown as nodules *in vitro*, Int. J. Cancer., (1994), 56, p731-735; Regulation of collagenase-3 expression in human breast carcinoma is mediated by stromal-epithelial cell interactions, Cancer Research (1997), 57, p4882-4888; Co-culture of human breast adenocarcinoma MCF-7 cells and human dermal fibroblasts enhances the production of matrix metalloproteinases 1,2 and 3 in fibroblasts, Brit.J.Cancer. (1995), 71, p1039-1045).

This invention describes the use of 3D microgravity culture to grow significant quantities of tumour cells and co-cultured cells for use as vaccines. We have found that it is possible to achieve the same biomass production in one 50 ml 3D microgravity vessel as is possible in 30 T175 flasks. By utilising a 500 ml 3D microgravity vessel we can achieve the same vaccine production of mammalian tumour cell lines as we could obtain by culturing 300 T175 flasks or 30 1,700 cm² roller bottles.

A further embodiment of this invention is the use of 3D co-culture to achieve a mixed cell type vaccine comprising an immortalised tumour cell line in combination with an immortalised or non-immortalised non-malignant cell line such as epithelial cells, stromal fibroblasts, bone stromal cells, endothelial cells, palisade cells or any other immortalised or non immortalised non-malignant cell type. Of particular interest are cells of the neuroendocrine lineage which are thought to have a paracrine function which may drive the progression of tumour development from hyperplasia to neoplastic to metastatic phenotypes. Two immortalised neuroendocrine tumour lines are known in the literature, the prostatic derived lung metastatic neuroendocrine line CRL5813 (NCI-H660) and the colorectal neuroendocrine line COLO320DMF, both of which secrete neuroendocrine factors such as parathyroid hormone, bombesin, etc. The cultures may be co-seeded with two or more cell types taken from independent 2D culture flasks, or the culture may be sequentially seeded for example with a stromal fibroblast first, followed some one or two days later by a tumour cell line.

A further aspect of this invention is a direct process of formulation of cells harvested from 3D microgravity culture without the need to treat the cells with trypsin. This process therefore has the advantage of significantly increasing the yield of cells following harvest, whereas for traditionally grown 2D cultures significant losses are incurred during the trypsinization, neutralization, washing and formulation. Cells harvested as spheroids tend to be significantly more robust to handle and wash, thereby leading to enhanced recoveries.

We also disclose an enhanced phenotype of a cancer vaccine resulting from growth as spheroids in 3D microgravity culture and more particularly co-cultured cells grown in 3D microgravity culture. Cells grown under 2D culture bear little resemblance to tumour cells *in vivo*. The monolayers of 2D grown cells have a uniform, cobblestone appearance, become contact inhibited, grow under conditions of optimal gas and nutrient supply and adhere directly to the plastic or glass vessel in which they are being grown. We have found that cells and more particularly co-cultures of two or more different cell types grown in 3D microgravity conditions grow in a way much more akin to tumour cell growth *in vivo*, single cell types and mixed cell types seeded directly into the microgravity chamber form spheroids which have an outer layer supplied optimally with gas and nutrients; possess an inner layer which is somewhat gas and nutrient deprived and slightly apoptotic; are adherent not to plastic or glass but to other cells and any extracellular matrix secreted by those cells; and which maintain a micro environment within the spheroid which may retain growth factors and other para- and autocrine secretions which would otherwise be diluted out by the media. This type of phenotype is advantageous for a cell line grown for use as a tumour vaccine to elicit a response against an existing tumour.

Analytical techniques such as immunohistochemistry, flow cytometry, two-dimensional electrophoresis, crossed immuno-electrophoresis, western blotting, DNA hybridization, fluorescent in situ hybridization and other techniques has revealed significant shifts in phenotype of established cell lines which result in significantly enhanced immunogenicity. A further embodiment of this invention is the use of mixed 3D microgravity culture incorporating non-malignant cell lines to enhance the phenotype of cell lines which hitherto have not been grown in mixed 3D culture.

A further embodiment of this invention is enhanced immunogenicity arising from the use of non-malignant cells in combination with tumour cell lines arising from direct presentation of peptides on the MHC-I molecules of the non-malignant cell line, particularly if the non-malignant cell line is matched or partially matched to the recipient. The peptides may be released from the tumour cell lines during growth, processing and irradiation of the 3D cell mass, or may be released during initial interaction of macrophages and dendritic cells at the time of injection.

A separate embodiment of this invention is the use of peptide extracts and other sub-cellular fractions derived from cells and co-cultures of cells grown under non-standard conditions. This includes cells grown on roller bottles, on microcarriers, on polyester fibre discs, 3D culture on non adherent flasks and 3D culture under microgravity conditions. The sub-cellular fractions or peptide fractions derived from the cells offer the opportunity to significantly increase the active component of a given vaccine. This aspect of the invention also allows for further fractionation of the sub-cellular fractions or peptides to select the active principles and to simplify the vaccine composition if so required.

A further embodiment of this invention is the production, composition and use of a mixed autologous / allogeneic cell vaccine comprising autologous non-malignant cells taken from an intended recipient of the vaccine grown in culture with an allogeneic immortalised tumour cell line. The autologous non-malignant cells have non-malignant levels of MHC-I and will thus have the capability of presenting in the context of autologous MHC-I peptides released from lysed, destroyed or apoptotic tumour cells with which they have been grown.

### Description of Drawings

**Figure 1** PSA expression analysis (PCR) of prostate cell iines grown in co-culture with bone marrow stromal cells
**Figure 2** Comparison of vaccine efficacy of *in vitro* grown K1735 cells and *in vivo* grown K1735 cells in a model of B16.F10 mouse melanoma
**Figure 3** Human CD4 T cell proliferative response to lysates of co-culture of DU-145 and NHBSF
**Figure 4** Human CD8 T cell proliferative response to lysates of co-culture of DU-145 and NHBSF
**Figure 5** Survival curves for rat therapy study utilising PA3 and PA3/YPEN/BMS co-cultures grown in 2D culture
**Figure 6** Comparative efficacy of 2D grown PA3 and PA3/YPEN/BMS co-cultures in rat protection study
**Figure 7** Comparative efficacy of 3D grown PA3 and PA3/YPEN/BMS co-cultures in rat protection study

### Examples

### Growth of a Murine Melanoma Cell Line with an Immortalised Fibroblast Line in 3D Culture

The murine melanoma cell line K1735 was grown with the murine fibroblast cell line 3T3 at a ratio of 1:1. The 3T3 cells were seeded at 5 x 10⁶ cells in a 50 ml cassette for a microgravity instrument (Synthecon) and allowed to grow for 2 days in DMEM 2% foetal calf serum, 10 mM glutamine. A further inoculum of 5x10⁶ cells of either K1735 was made into the 50 ml rotating cassette (Synthecon) and the culture inoculated for a further 6-9 days. The resulting spheroids were harvested and washed by light centrifugation in DMEM at day 8-11 and taken for further analysis.

A comparison was made between the 3D co-cultured 3T3 / K1735 and a mixture of 3T3 and K1735 cells grown in separate 2D cultures and mixed post harvest. The cells were trypsinized and the mixture of cells from 2D and 3D cultures were analyzed for the level of apoptosis by annexin and propidium iodide staining and also for phenotypic expression of various markers. Further analysis of small immunogenic peptides extracted by the method of Nair et al (1997 Eur. J. Immunol. 27, p589-597) was performed using Surface Enhanced Laser Desorption lonisation (SELDI) mass spectrometry. The 3D co-cultured fibroblasts and melanoma cells produce a significantly more complex pattern of peptides some of which are unique to the co-culture system.

Growth of a Human Prostatic Tumour Cell Line with a Human Prostatic Non-malignant Epithelial Cell Line.

The human prostatic epithelial tumour line NIH1542 was grown with the non-malignant human prostatic epithelial line PNT2-C2 at a 1:1 ratio in a 3D microgravity culture vessel (Synthecon). The cell lines were co-inoculated at 5x10⁶ cells each and cultured in KSFM for a period of 6-11 days until a significant biomass of spheroids had accumulated. The cell mass was harvested and washed in serum free medium and then trypsinized to prepare the sample for analysis.

A comparison was made between the 3D co-cultured NIH-1542 / PNT2-C2 and a mixture of NIH-1542 and PNT2-C2 cells grown in separate 2D culture and mixed post harvest. The cells were trypsinized and the mixture of cells from 2D and 3D cultures were analyzed for the level of apoptosis by annexin and propidium iodide staining and also for phenotypic expression of various markers. Further analysis of small immunogenic peptides extracted by the method of Nair et al (1997 Eur. J. Immunol. 27, p589-597) was performed using Surface Enhanced Laser Desorption lonisation (SELDI) mass spectrometry. The 3D co-cultured fibroblasts and melanoma cells produce a significantly more complex pattern of peptides some of which are unique to the co-culture system.

### Growth of Human Prostatic Tumour Cell Line with a Human Prostatic Derived Neuroendocrine Lung Metastasis Cell Line.

The human prostatic epithelial tumour line NIH1542 was grown with the human prostatic neuroendocrine epithelial line CRL-5813 at a 1:1 ratio in a 3D microgravity culture vessel (Synthecon). The cell lines were co-inoculated at 5x10⁶ cells each and cultured in KSFM for a period of 6-11 days until a significant biomass of spheroids had accumulated. ). In parallel two separate 2D cultures were seeded and grown to near confluence. The cells were trypsinized and the mixture of cells from 2D and 3D co-culture were analyzed for the level of apoptosis by annexin and propidium iodide staining and. also for phenotypic expression of various markers. Further analysis of small immunogenic peptides extracted by the method of Nair et al (1997 Eur. J. Immunol. 27, p589-597) was performed using Surface Enhanced Laser Desorption lonisation (SELDI) mass spectrometry. The 3D co-cultured epithelial and neuroendocrine cells produce a significantly more complex pattern of peptides than the mixed 2D grown cells, some of which are unique to the co-culture system.

### Production of a vaccine comprising a Murine Melanoma Cell Line with an Immortalised Fibroblast Line in 3D Culture

The murine melanoma cell line K1735 was grown with the murine fibroblast cell line 3T3 at a ratio of 1:1. The 3T3 cells were seeded at 5 x 10⁶ cells in a 50 ml cassette for a microgravity instrument (Synthecon) and allowed to grow for 2 days in DMEM 2% foetal calf serum, 10 mM glutamine. A further inoculum of 5x10⁶ cells of either K1735 was made into the 50 ml rotating cassette (Synthecon) and the culture inoculated for a further 6-9 days.

The cells were allowed to grow for a period of 7-14 days with intermediate media and growth factor replenishment. At harvest the cassette was emptied into a centrifuge vessel and lightly centrifuged to pellet the biomass. The visible and invisible spheroids were washed three times in PBS or other inert media, irradiated at 50-300 Gy and then either used directly in the protection experiment or; formulated in appropriate freezing mixture (10% DMSO in Hanks) and aliquoted on the basis of either cell number determined by trypsinization and counting or on a protein or nucleic acid basis. No trypsinization was required to remove the vaccine cells from the culture vessel thereby retaining valuable TAA and TSA which will elicit an anti-tumour response in the recipient mammal. Furthermore the spheroids are significantly easier to harvest as a result of their size, requiring less aggressive centrifugation than is normally required for individual cells which leads to significant enhancement of the recovered cell mass.

An aliquot of co-cultured spheroids (1-10 x 10⁶ cells) were used in a standard model of allogeneic tumour protection in the B16F10 tumour model.

Groups of 8 C57/B6 mice were immunized with 1-10 x10⁶ K1735 / 3T3 spheroids and compared to 2D flask grown K1735 cells irradiated at 150 Gy, at T-10 days. The mice were then challenged on Day 0 with 5x10⁵ live B16F10 cells and then monitored for tumour growth.

### PSA expression in prostate cell lines grown alone and in co-culture with normal bone marrow stromal cells

### Methodology

### Cell culture

Cell lines ONYCAP-1, ONYCAP-23, P4E6 and SHMAC-4 were seeded at 1 x 10⁶ cells onto confluent monolayers of NHBMSC cells in T175 culture flasks. Co-cultures were maintained for 2 days using keratinocyte growth medium supplemented with 5% foetal calf serum. Cells were harvested by scraping from the surface of the plastic and washed in Hanks balanced salt solution before RNA extraction.

Cell lines designated Onycap1 and Onycap 23 have been deposited under the Budapest Treaty at ECACC on 28 March 2000 under the Accession numbers 00032802 and 00032801 respectively.

### RNA extraction

A double extraction was performed using TRI REAGENT (Sigma #T9424). The reagent was added directly to the washed cell pellets and samples were allowed to stand for 5 minutes before the addition of chloroform. Samples were vortexed and allowed to stand for a further 10 minutes at room temperature then centrifuged at 12,000xg for 15 minutes.

The upper aqueous phase was transferred to a fresh tube, another aliquot of TRI REAGENT added and the above steps were repeated for the second stage extraction. The aqueous phase was again transferred to a fresh tube and the RNA precipitated with isopropanol. RNA pellets were washed with 75% ethanol, dried and re-suspended in TE buffer.

### DNase treatment

An aliquot of each RNA sample was treated with Deoxyribonuclease I (Life Technologies #18068-015) to ensure there was no contamination with genomic DNA. Reactions were incubated for 15 minutes at room temperature then the DNase inactivated by the addition of 25mM EDTA and heating to 65°C for 10 minutes.

### Reverse Transcription

Reverse transcription was performed using the 1st strand cDNA synthesis kit for RT-PCR (AMV) from Boehringer Mannheim (#1 483 188). Reactions were incubated at 25°C for 10 minutes then at 42°C for 1 hour. The AMV enzyme was denatured by heating to 99°C for 5 minutes then the reaction was cooled to 4°C.

### PCR

PCR primers were designed from published sequences. Verification of cDNA integrity was achieved by PCR amplification using GapdH housekeeping primers. Amplification reactions with the external primer set were carried out using thermocycler program (1). The internal nested PCR set was run on program (2) below.

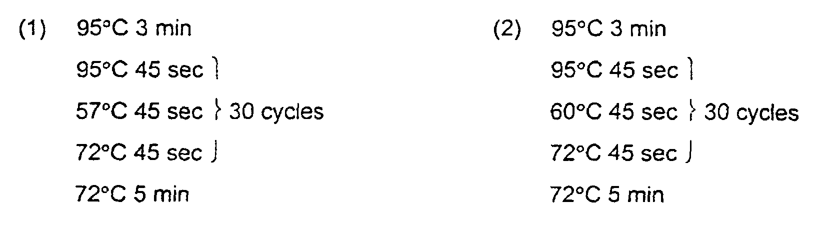

Positive controls included cDNA from LnCAP. Negative controls included no cDNA template. Table of tumour antigen PCR primer sets

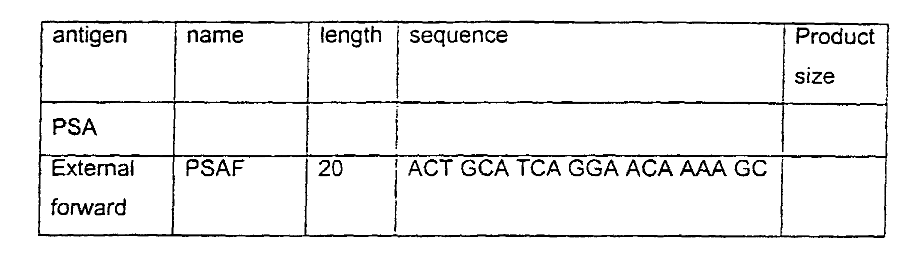

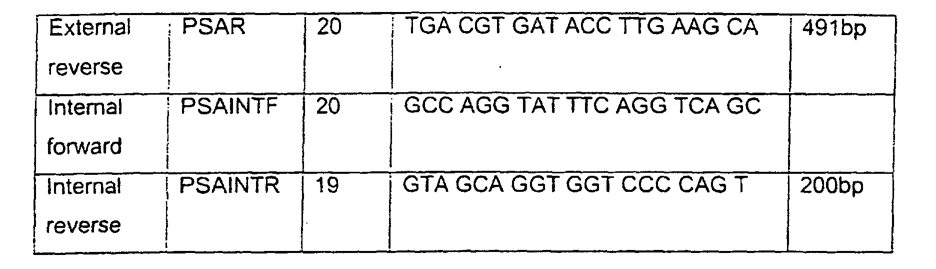

### Results

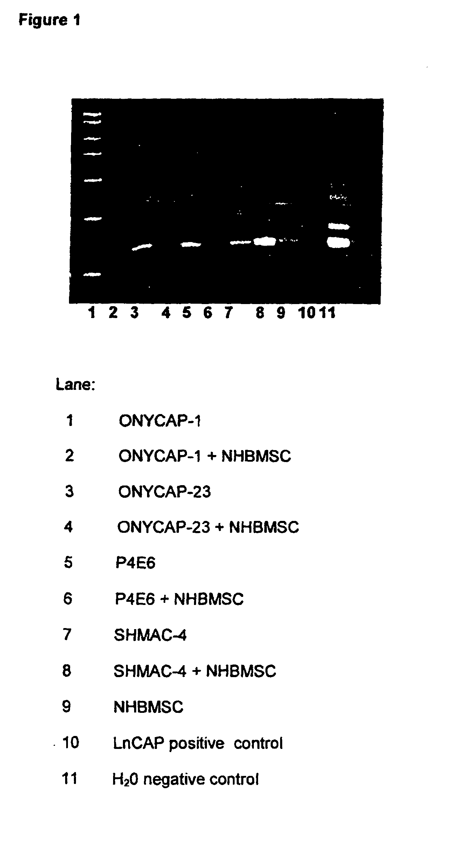

The PCR samples were run in pairs. The prostate cells alone then the same cells grown in co-culture with NHBMSC followed by NHBMSC alone then a positive and negative control Figure 1.

PSA expression was not detected in 3 out of 4 prostate cells grown alone however, cDNA isolated from all of the cell lines grown in co-culture with NHBMSC produced a band at 200bp following 2 rounds of PCR amplification suggesting an up-regulation of PSA as a result of co-culture with bone marrow stromal cells.

### Antigen expression in prostate cell lines grown alone and in co-culture with normal bone marrow stromal cells

### Methodology

### Cell culture

Cell line ONYCAP-23 were seeded at 1 x 10⁶ cells onto confluent monolayers of NHBMSC cells in T175 culture flasks. Co-cultures were maintained for 2 days using keratinocyte growth medium supplemented with 5% foetal calf serum. Cells were harvested by scraping from the surface of the plastic and washed in Hanks balanced salt solution before RNA extraction.

### RNA extraction

A double extraction was performed using TRI REAGENT (Sigma #T9424). The reagent was added directly to the washed cell pellets and samples were allowed to stand for 5 minutes before the addition of chloroform. Samples were vortexed and allowed to stand for a further 10 minutes at room temperature then centrifuged at 12,000xg for 15 minutes.

The upper aqueous phase was transferred to a fresh tube, another aliquot of TRI REAGENT added and the above steps were repeated for the second stage extraction. The aqueous phase was again transferred to a fresh tube and the RNA precipitated with isopropanol. RNA pellets were washed with 75% ethanol, dried and re-suspended in TE buffer.

### DNase treatment

An aliquot of each RNA sample was treated with Deoxyribonuclease I (Life Technologies #18068-015) to ensure there was no contamination with genomic DNA. Reactions were incubated for 15 minutes at room temperature then the DNase inactivated by the addition of 25mM EDTA and heating to 65°C for 10 minutes.

### Reverse Transcription

Reverse transcription was performed using the 1st strand cDNA synthesis kit for RT-PCR (AMV) from Boehringer Mannheim (#1 483 188). Reactions were incubated at 25°C for 10 minutes then at 42°C for 1 hour. The AMV enzyme was denatured by heating to 99°C for 5 minutes then the reaction was cooled to 4°C.

### PCR

PSA PCR primers were designed from published sequences tabulated below. Verification of cDNA integrity was achieved by PCR amplification using GapdH housekeeping primers. Amplification reactions with the external primer set were carried out using thermocycler program (1). The internal nested PCR set was run on program (2) below.

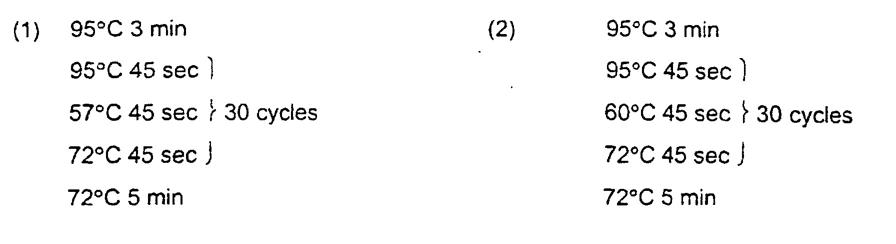

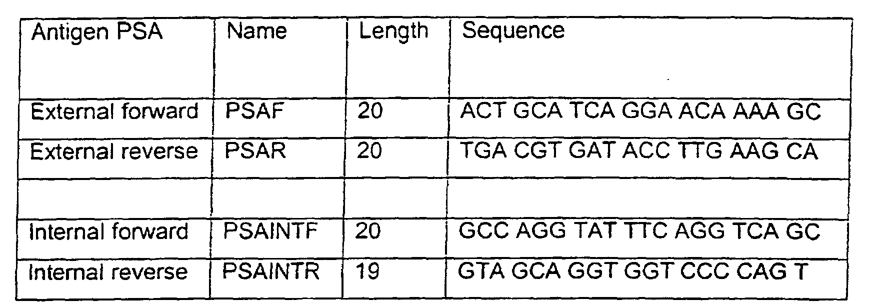

Androgen receptor, Hyaluronic acid receptor and MMP-9 PCR primers were designed from published sequences tabulated below. Verification of cDNA integrity was achieved by PCR amplification using GapdH housekeeping primers. Amplification reactions with the external primer set were carried out using thermocycler program are given below and were run on the thermocylcer

### Antigen Primer Sets

Androgen Receptor

Forward primer GTC CAA GAC CTA CCG AGG AG

Reverse primer CTG CTG TTG CTG AAG GAG TT

MMP-9

Forward primer AGA ACC AAT CTC ACC GAC AG

Reverse primer AGG GAC CAC AAC TCG TCA T

Hyaluronic acid receptor

Forward primer CTG GGT TGG AGA TGG ATT C

Reverse primer CAA AGA AGA GGA GAG CAA GC

### Amplification conditions

| Stage | Temperature | Time | No. of cycles |
|---|---|---|---|
| 1 | 94°C | 3 minutes | 1 |
| | | | |
| 2 | 94°C | 45 seconds | |
| | 57°C | 45 seconds | 30 |
| | 72°C | 45 seconds | |
| | | | |
| 3 | 72°C | 5 minutes | 1 |

### Results

The PCR samples were run in pairs on agarose gels. The prostate cells alone then the same cells grown in co-culture with NHBMSC.

The results are tabulated in Table 1 where + indicates the presence of relevant PCR product at the expected molecular weight and - indicates the absence of a relevant PCR product. Clearly the co-culture of Onycap-23 with NHBSC has resulted in the expression of mRNA for significant prostate and metastatic antigens whereas the basal NHBSC and Onycap23 have not produced any of these significant antigens.

### In Vivo Growth of Tumour Produces a More Efficacious Vaccine

Growth of a tumour in vivo elicits a massive stromal reaction whereby adjacent tissue including epithelial cells, stromal fibroblasts, blood vessel endothelial cells grow into the tumour mass. There is abundant evidence in the literature that tumour elicited stroma shows aberrant expression of proteins in response to the tumour cells.

In this example we examine the potential of using an in situ grow tumour with attendant stroma to determine if inclusion of stroma cells in a vaccine, whether that be epithelial, fibroblast or endothelial, offers an advantage over tumour cells alone.

We compared in vitro grown K1735 mouse melanoma cells with K1735 grown in its syngeneic host C3H mice in a model of vaccination therapy against a B16.F10 melanoma tumour.

K1735 (P25) cells were grown in RPMI 1640 medium supplemented with 10% FCS and 2 mM L-glutamine, trypsinised, irradiated at 100 Gy and formulated into 10%DMSO in RPMI at a cell density of 5x106 cells / ml prior to cryopreservation at -1800C.

A K1735 tumour grown in a C3H mouse was excised and chopped into small pieces before being forced through a 70um gauze, irradiated at 100 Gy and formulated in 10%DMSO in RPMI at a cell density of 5x106 cells / ml, then cryopreserved at -1800C.

B16.F10 mouse melanoma clone G10 was grown in RPMI 1640 medium supplemented with 10% FCS and 2 mM L-glutamine, trypsinised and used to inoculate groups of 5 C57BL6 mice at a viable dose of 1x104 cells in 200ul of medium. Three days following viable tumour challenge the mice were dosed with 1x106 of either in vitro grown K1735 or in vivo grown tumour or PBS injected mice and subsequently on three other occasions on days 6 10 and 13 post tumour challenge. Tumour volume and survival were measured (sacrifice when tumour attained 15x15mm) at three day intervals starting at day 11 post tumour challenge.

### Results

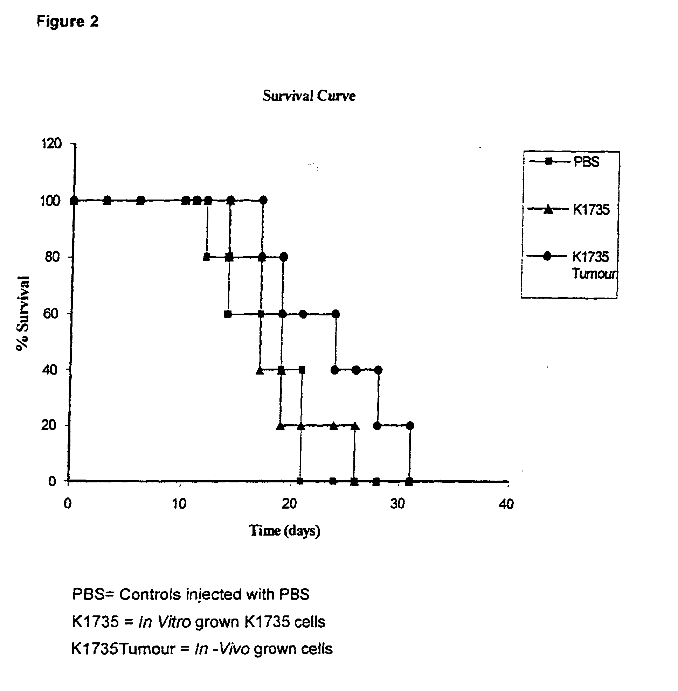

A significant increase in survival attendant with slower rate of tumour growth was observed in the group vaccinated with in vivo grown K1735 when compared to in vitro grown K1735 (Figure 2). The number of *in vivo* grown K1735 cells used to vaccinate will also be considerably be less since the vaccine was formulated on a total number basis and not specifically a K1735 number basis. Therefore since we observe a massive stromal involvement in *in vitro* grown cells the actual number of K1735 cells in the tumour cell suspension prepared for the vaccine will be relatively low and thus the significant improvement in vaccine potency is due to the non-tumour stromal cells accompanying the K1735 melanoma cells.

### Human Prostate Cells Grown on Prostate Stromal Fibroblasts Show Altered Gene Expression.

### Methodology

### Cell culture

Cell line DU145 was seeded at 1 x 10⁶ cells onto confluent monolayers of normal human prostatic stromal fibroblasts (NHPSF) in T175 culture flasks. Co-cultures were maintained for 2 days using in MEM medium supplemented with 10% FCS and 2 mM L-glutamine. Cells were harvested by scraping from the surface of the plastic and washed in Hanks balanced salt solution before RNA extraction.

Normal human prostatic stromal fibroblasts (NHPSF) were grown to confluency in a T175 flask in MEM medium supplemented with 10% FCS and 2 mM L-glutamine.

Cells were harvested by scraping from the surface of the plastic and washed in Hanks balanced salt solution before RNA extraction.

Cell line DU145 was seeded at 1 x 10⁶ cells into T175 flasks and grown in MEM medium supplemented with 10% FCS and 2 mM L-glutamine and grown to confluency. Cells were harvested by scraping from the surface of the plastic and washed in Hanks balanced salt solution before RNA extraction.

### RNA extraction

A double extraction was performed using TRI REAGENT (Sigma #T9424). The reagent was added directly to the washed cell pellets and samples were allowed to stand for 5 minutes before the addition of chloroform. Samples were vortexed and allowed to stand for a further 10 minutes at room temperature then centrifuged at 12,000xg for 15 minutes.

The upper aqueous phase was transferred to a fresh tube, another aliquot of TRI REAGENT added and the above steps were repeated for the second stage extraction. The aqueous phase was again transferred to a fresh tube and the RNA precipitated with isopropanol. RNA pellets were washed with 75% ethanol, dried and re-suspended in TE buffer.

### DNase treatment

An aliquot of each RNA sample was treated with Deoxyribonuclease I (Life Technologies #18068-015) to ensure there was no contamination with genomic DNA. Reactions were incubated for 15 minutes at room temperature then the DNase inactivated by the addition of 25mM EDTA and heating to 65°C for 10 minutes.

### DNA Array Probing

Labelled cDNA was used to probe a Clontech Atlas™ array (human cancer array II) following the manufacturers protocol for hybridisation and washing. Images were recorded utilising a phosphor imager and recorded files analysed with the Clontech Atlas Image™ software.

### Results

By equalising the amounts of cDNA used to probe the DNA arrays we were able to make an assessment of the gene expression in the co-culture and compare it to the same gene expression in the individual cell lines. Chung *et al* noted by morphological and growth rate observations that prostate tumour cell lines co-cultured with prostatic fibroblasts were actually inhibited and held in check. We observe in Table 2 that many genes are down regulated as a result of growth on prostate derived stromal fibroblasts but that in addition a small number are up-regulated Table 3.

This methodology allows for the identification of genes which are up or down regulated by growth of tumour cell lines on stromal cells from various organ compartments. By choosing tumour cell lines with varying degrees of invasivity or metastatic capability in combination with stromal cells such as prostatic fibroblasts, bone stromal fibroblasts, or endothelial cells it is possible to identify significant genes which are temporally and spatially expressed as a result of the intimate contact between tumour and stromal cell and thus enable the identification of antigens which are thus meaningful and viable potential targets for immunotherapy or other therapeutic strategies.

### Human Prostate Cells Grown on Bone Stromal Fibroblasts Show Altered Gene Expression.

### Methodology

### Cell culture

Cell line DU145 was seeded at 1 x 10⁶ cells onto confluent monolayers of normal human bone stromal fibroblasts (NHBSF) in T175 culture flasks. Co-cultures were maintained for 2 days using in MEM medium supplemented with 10% FCS and 2 mM L-glutamine. Cells were harvested by scraping from the surface of the plastic and washed in Hanks balanced salt solution before RNA extraction.

Normal human bone stromal fibroblasts (NHBSF) were grown to confluency in a T175 flask in MEM medium supplemented with 10% FCS and 2 mM L-glutamine. Cells were harvested by scraping from the surface of the plastic and washed in Hanks balanced salt solution before RNA extraction.

Cell line DU145 was seeded at 1 x 10⁶ cells into T175 flasks and grown in MEM medium supplemented with 10% FCS and 2 mM L-glutamine and grown to confluency. Cells were harvested by scraping from the surface of the plastic and washed in Hanks balanced salt solution before RNA extraction.

### RNA extraction

A double extraction was performed using TRI REAGENT (Sigma #T9424). The reagent was added directly to the washed cell pellets and samples were allowed to stand for 5 minutes before the addition of chloroform. Samples were vortexed and allowed to stand for a further 10 minutes at room temperature then centrifuged at 12,000xg for 15 minutes.

The upper aqueous phase was transferred to a fresh tube, another aliquot of TRI REAGENT added and the above steps were repeated for the second stage extraction. The aqueous phase was again transferred to a fresh tube and the RNA precipitated with isopropanol. RNA pellets were washed with 75% ethanol, dried and re-suspended in TE buffer.

### DNase treatment

An aliquot of each RNA sample was treated with Deoxyribonuclease I (Life Technologies #18068-015) to ensure there was no contamination with genomic DNA. Reactions were incubated for 15 minutes at room temperature then the DNase inactivated by the addition of 25mM EDTA and heating to 65°C for 10 minutes.

### Reverse Transcription

Reverse transcription was performed using the 1st strand cDNA synthesis kit for RT-PCR (AMV) from Boehringer Mannheim (#1 483 188). Reactions were incubated at 25°C for 10 minutes then at 42°C for 1 hour. The AMV enzyme was denatured by heating to 99°C for 5 minutes then the reaction was cooled to 4°C. 32P or 33P deoxynucleotides were used to label the cDNA for subsequent probing of the arrays. RNA from DU145 and NHBSF cultures were mixed in equal proportions and the same quantity of RNA used in the reverse transcription as was used for the co-culture RNA.

### DNA Array Probing

Labelled cDNA was used to probe a Clontech Atlas™ array (human array II) following the manufacturers protocol for hybridisation and washing. Images were recorded utilising a phosphor imager and recorded files analysed with the Clontech Atlas Image™ software.

### Results

By equalising the amounts of cDNA used to probe the DNA arrays we were able to make an assessment of the gene expression in the co-culture and compare it to the same gene expression in the individual cell lines by mixing cDNA samples from the individual prostate tumour cells and NHBSF. Chung *et al* noted by morphological and growth rate observations that prostate tumour cell lines co-cultured with prostatic fibroblasts were actually inhibited and held in check. We observe in Table 4 that many genes are down regulated as a result of growth on prostate derived stromal fibroblasts but that in addition a small number are up-regulated Table 5.

This methodology allows for the identification of genes which are up or down regulated by growth of tumour cell lines on stromal cells from various organ compartments. By choosing tumour cell lines with varying degrees of invasivity or metastatic capability in combination with stromal cells such as prostatic fibroblasts, bone stromal fibroblasts, or endothelial cells it is possible to identify significant genes which are temporally and spatially expressed as a result of the intimate contact between tumour and stromal cell and thus enable the identification of antigens which are thus meaningful and viable potential targets for immunotherapy or other therapeutic strategies.

### Human T Cell Proliferative Response to Lysates of Co-Culture of DU-145 and NHBSF

### Methodology

We performed a proliferation assay on T-cells following stimulation with lysates of the prostate cell lines to determine if patients vaccinated with prostate cell lines resulted in a specific expansion of T-cell populations that recognised antigens derived from the vaccinating cell lines and also a co-culture of DU-145 and NHBSF. Whole blood was extracted at each visit to the clinic and used in a BrdU (bromodeoxyuridine) based proliferation assay as described below:

### Patient BrdU proliferation method

### Reagents

| | | |
|---|---|---|
| RPMI | | Life Technologies, Paisley, Scotland. |
| BrdU | | Sigma Chemical Co, Poole, Dorset. |
| PharMlyse | 35221 E | Pharmingen, Oxford UK |
| Cytofix/Cytoperm | 2090KZ | " |
| Perm/Wash buffer (x10) | 2091 KZ | " |
| FITC Anti-BrdU/Dnase | 340649 | Becton Dickinson |
| PerCP Anti-CD3 | 347344 | " |
| Pe Anti-CD4 | 30155X | Pharmingen |
| Pe Anti-CD8 | 30325X | " |
| FITC mu-IgG1 | 349041 | Becton Dickinson |
| PerCP IgG1 | 349044 | " |
| PE IgG1 | 340013 | " |

### Method

1) Dilute 1 ml blood with 9 ml RPMI + 2mM L-gln +PS +50µM 2-Me. Do not add serum. Leave overnight at 37°C
2) On following morning, aliquot 450µl of diluted blood into wells of a 48-well plate and add 50µl of stimulator lysate. The lysate is made by freeze-thawing tumour cells (2x10⁶ cell equivalents/ml) x3 in liquid nitrogen and then storing aliquots frozen until required.
3) Culture cells at 37°C for 5 days
4) On the evening of day 5 add 50µl BrdU @ 30µg/ml
5) Aliquot 100µl of each sample into a 96-well round-bottomed plate.
6) Spin plate and discard supernatant
7) Lyse red cells using 100µl *Pharmlyse* for 5minutes at room temperature
8) Wash x2 with 50µl of Cytofix
9) Spin and remove supernatant by flicking
10) Permeabilise with 100µl Perm wash for 10mins at RT
11) Add 30µl of antibody mix comprising antibodies at correct dilution made up to volume with Perm-wash
12) Incubate for 30 mins in the dark at room temperature.
13) Wash x1 and resuspend in 100µl 2% paraformaldehyde
14) Add this to 400µl FACSFlow in cluster tubes ready for analysis
15) Analyse on FACScan, storing 3000 gated CD3 events.

### 6-well plate for stimulation

| | Nil | ConA | 1542 | LnCap | Du145 | Pnt2 |
|---|---|---|---|---|---|---|
| PBL 1 | | | | | | |
| PBL 2 | | | | | | |
| PBL 3 | | | | | | |
| PBL 4 | | | | | | |
| PBL 5 | | | | | | |
| PBL 6 | | | | | | |

### 96-well plate for antibody staining

| PBL 1 | | PBL 2 | | PBL 3 | | PBL 4 | | PBL 5 | | PBL 6 | |
|---|---|---|---|---|---|---|---|---|---|---|---|
| Nil A | 15 D | Nil A | 15 D | Nil A | 15 C | Nil A | 15 D | Nil A | 15 D | Nil A | 15 D |
| Nil D | 15 E | Nil D | 15 E | Nil D | 15 E | Nil D | 15 E | Nil D | 15 E | Nil D | 15 E |
| Nil E | Ln D | Nil E | Ln D | Nil E | Ln C | Nil E | Ln D | Nil E | Ln D | Nil E | Ln D |
| Con D | Ln E | Con D | Ln E | Con D | Ln E | Con D | Ln E | Con D | Ln E | Con D | Ln E |
| Con E | Du D | Con E | Du D | Con E | Du D | Con E | Du D | Con E | Du D | Con E | Du D |
| | Du E | | Du E | | Du E | | Du E | | Du E | | Du E |
| | Pn D | | Pn D | | Pn D | | Pn D | | Pn D | | Pn D |
| | Pn E | | Pn E | | Pn E | | Pn E | | Pn E | | Pn E |
| **Legend:** | | | | | | | | | | | |
| A: IgG1-FITC (5µl) IgG1-PE (5µl) IgG1-PerCP (5µl) 15µlMoAb+15µl | | | | | | | | | | | |
| D: BrdU-FITC (5µl) CD4-PE (5µl) CD3-PerCP (5µl) 15µlMoAb+15µl | | | | | | | | | | | |
| E: BrdU-FITC (5µl) CD8-PE (5µl) CD3-PerCP (5µl) 15µlMoAb+15µl | | | | | | | | | | | |
| 15: NIH1542-CP3TX | | | | | | | | | | | |
| Ln: LnCap | | | | | | | | | | | |
| D: Du145 | | | | | | | | | | | |
| Pn: PNT2 | | | | | | | | | | | |
| Con: ConA lectin (positive control) | | | | | | | | | | | |
| Nil: No stimulation | | | | | | | | | | | |

### Results

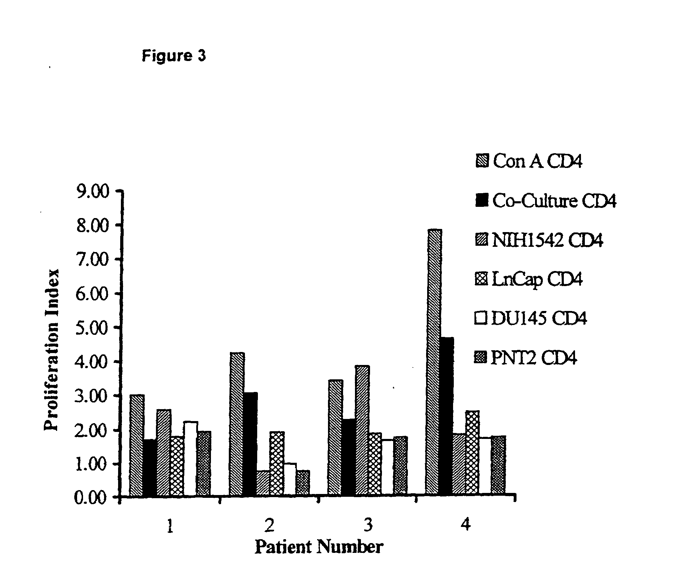

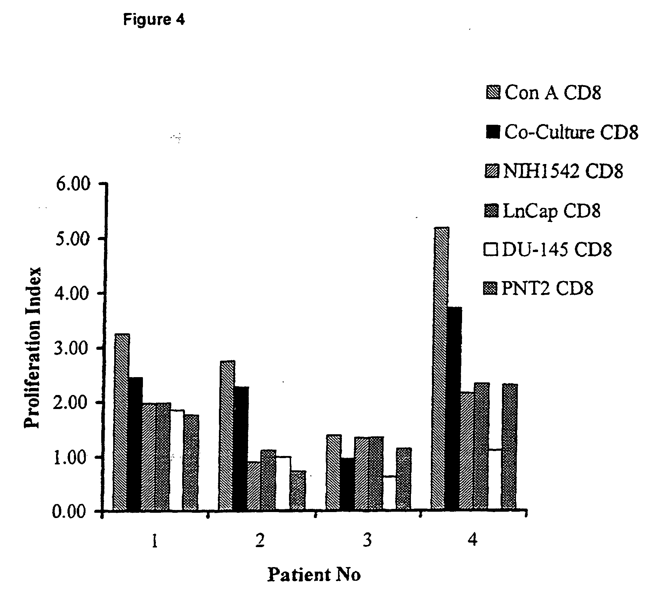

The results for the proliferation assays are shown in where a proliferation index for either CD4 (Figure 3) or CD8 (Figure 4) positive T-cells are plotted against the various cell lysates, the proliferation index being derived by dividing through the percentage of T-cells proliferating by the no-lysate control.

Results are shown for patient numbers 1, 2, 3, and 4 who had received a series of vaccinations with human prostate cell lines, PNT-2, NIH1542, DU145 and LnCap in a clinical trial to evaluate the safety and immunogenicity of these cell lines. Results are given for cell lysates namely, NIH1542, LnCap, DU-145, PNT-2 and also a co-culture of DU145 and NHBSF celis in such a way that the same cell number equivalent is used in each proliferation assay. In this heterogeneous population of patients vaccinated with a variety of cell lines it is interesting to note that the co-culture lysate has led to a significant improvement in CD4 and CD8 proliferative response in 3 out of 4 patients. This is indicative of a more representative spectrum of antigens being produced in co-culture than in the mono-culture of the DU-145 alone.

### 2D Co-Culture of Prostate Tumour Line with Bone Stromal Cells and Endothelial Cells Enhances Vaccine Efficacy

The rat PA3 prostate tumour line is a relatively weak allogeneic immunogen in the copenhagen rat challenged with autologous MatLyLu prostate cells. We compared PA3 rat prostate tumour cells grown in T185 flasks and PA3 cells co-cultured with bone stromal fibroblasts and YPEN endothelial cells in T185 flasks as a vaccine in a protection model.

### PA3 Clone 1 2D

PA3 Clone 1 cells (1 x 10⁶) cells were seeded into a T175 tissue culture flask in 35 ml of RPMI 1640 culture medium supplemented with 10% FCS/2 mM Glutamine.
Cells were allowed to grow until confluent under conditions of 37°C/5% CO₂. Confluent cells were harvested by washing the monolayer with 10ml Hank's buffered salt solution, followed by 5ml trypsin/EDTA solution to remove cells from the flask. Once all cells had lifted away from the flask surface the trypsin reaction was stopped by the addition of 5ml FCS. The 10ml of cell suspension was collected in a 50ml tube, centrifuged at 18,000 rpm for 3 minutes. The resultant pellet was re-suspended into 10ml PBS and stored on ice, prior to irradiation

### PA3/YPEN 2D Co-Culture

PA3 Clone 1 cells (1 x 10⁵) and YPEN cells (1 x 10⁵) were seed simultaneously into a T75 tissue culture flask containing 15ml RPMI 1640 culture medium supplemented with 10% FCS/2mM Glutamine. Cells were allowed to grow until confluent and harvested by cell scraping, pelleted by centrifugation at 18,000 rpm for 3 min, and re-suspended in 10ml PBS. Cells were stored on ice prior to irradiation.

### PA3 / YPEN / RBMS 2D Co-Culture

PA3 Clone 1 cells (1 x 10⁵), YPEN cells 1 x 10⁵ and RBMS (1 x 10⁵) were seeded simultaneously into a T75 tissue culture flask containing 15ml RPMI 1640 culture medium supplemented with 10% FCS/2mM Glutamine. Cells were allowed to grow until confluent and harvested by cell scraping, pelleted by centrifugation at 18,000 rpm for 3 min, and re-suspended in 10ml PBS. Cells were stored on ice prior to irradiation.

### PA3 / RBMS 2D Co-Culture

PA3 Clone 1 cells (1 x 105) and (1 x 105) RBMS were seeded simultaneouslyinto a T75 tissue culture flask containing 15ml RPMI 1640 culture medium supplemented with 10% FCS/2mM Glutamine. Cells were allowed to growuntil confluent and harvested by cell scraping, pelleted by centrifugation at 18,000 rpm for 3 min, and re-suspended in 10ml PBS. Cells were stored once prior to irradiation.

### Irradiation and Cell Count.

Tubes containing cell suspensions were irradiated with 6 x 25 Gy (Total: 150 Gy). A cell count is then performed on each sample using a haemocytometer. Briefly 10µl of each cell suspension in mixed with 90µl of tryphan blue and approx. 10µl was loaded under the coverslip of the haemocytometer. A cell count was performed using a x20 objective lens. Each cell suspension was then centrifuged at 18,000 rpm for 3 minutes. The resultant pellet was re-suspended into vaccine freezing medium (RPMI 1640/8%FCS/8%DMSO) at 5 x 10⁶ cells per ml of freezing medium. Suspensions were then aliquoted into cryovials at 1.2ml per vial; vials were then placed into a -80°C freezer overnight prior to storage in liquid nitrogen.

### Injection Protocol (vaccine)

The appropriate vials were removed from storage in liquid nitrogen, thawed and 0.2 ml from the vial was injected into shaved right hind flank of a copenhagen rat. 1 vial is sufficient to inject 5 animals (1 experimental group). See below for injection regime for protection and therapy

### Culture of 'Challenge' Tumor Cell Line

### The challenge material used for these experiments is the cell line Mat-Ly-Lu (Wild Type)

Mat-Ly-Lu cells (1 x 10⁶) were seeded into a T175 tissue culture flask in 35 ml of RPMI 1640 culture medium supplemented with 10% FCS/2 mM Glutamine. Cells were allowed to grow until confluent under conditions of 37°C/5% CO₂. Confluent cells were harvested by washing the monolayer with 10ml Hank's buffered salt solution, followed by 5ml trypsin/EDTA solution to remove cells from the flask. Once all cells had lifted away from the flask surface the trypsin reaction was stopped by the addition of 5ml FCS. The 10ml of cell suspension was collected in a 50ml tube, centrifuged at 18,000 rpm for 3 minutes. The resultant pellet was re-suspended into 10ml PBS and stored on ice. A cell count was performed as detailed above and cell suspension adjusted to 5 x 10⁵ per ml and aliquoted into cryovials at 1.2 ml per vial. Vials were then held on ice prior to injection.

### Injection Protocol (challenge)

Irradiated Mat-Ly-Lu cells (0.2 ml) were injected into shaved left hind flank of a copenhagen rat. 1 vial is sufficient to inject 5 animals (1 experimental group). See below for injection regime for protection and therapy

### Therapy Model

| | **Day 0** **Challenge** | **Day 2** **Therapy** | **Day 7** **Therapy** | **Day 10** **Therapy** |
|---|---|---|---|---|
| Group 1 | WT MLL 1 x 10⁵ per Rat | Freezing Medium | Freezing Medium | Freezing Medium |
| Group 2 | WT MLL 1 x 10⁵ per Rat | PA3 Clone 1 2D 1 x 10⁶ per Rat | PA3 Clone 1 2D 1 x 10⁶ per Rat | PA3 Clone 1 2D 1 x 10⁶ per Rat |
| Group 3 | WT MLL 1 x 10⁵ per Rat | PA3 Clone 1 + YPEN 2D 1 x 10⁶ per Rat | PA3 Clone 1 + YPEN 2D 1 x 10⁶ per Rat | PA3 Clone 1 + YPEN 2D 1 x 10⁶ per Rat |
| Group 4 | WT MLL 1 x 10⁵ per Rat | PA3 Clone 1 + YPEN + RBMS 2D 1 x 10⁶ per Rat | PA3 Clone 1 + YPEN + RBMS 2D 1 x 10⁶ per Rat | PA3 Clone 1 + YPEN + RBMS 2D 1 x 10⁶ per Rat |
| Group 5 | WT MLL 1 x 10⁵ per Rat | PA3 Clone 1 + RBMS 2D 1 x 10⁶ per Rat | PA3 Clone 1 + RBMS 2D 1 x 10⁶ per Rat | PA3 Clone 1 + RBMS 2D 1 x 10⁶ per Rat |

### Protection Model

| | **Day -14** **Protection** | **Day -7** **Protection** | **Day 0** **Challenge** |
|---|---|---|---|
| Group 1 | Freezing Medium | Freezing Medium | WT MLL 1 x 10⁵ per Rat |
| Group 2 | PA3 Clone 1 2D 1 x 10⁶ per Rat | PA3 Clone 1 2D 1 x 10⁶ per Rat | WT MLL 1 x 10⁵ per Rat |
| Group 3 | PA3 Clone 1 + YPEN 2D 1 x 10⁶ per Rat | PA3 Clone 1 + YPEN 2D 1 x 10⁶ per Rat | WT MLL 1 x 10⁵ per Rat |
| Group 4 | PA3 Clone 1 + YPEN + RBMS 2D 1 x 10⁶ per Rat | PA3 Clone 1 + YPEN + RBMS 2D 1 x 10⁶ per Rat | WT MLL 1 x 10⁵ per Rat |
| Group 5 | PA3 Clone 1 + RBMS 2D 1 x 10⁶ per Rat | PA3 Clone 1 + RBMS 2D 1 x 10⁶ per Rat | WT MLL 1 x 10⁵ per Rat |

### Animal Measurement Criteria

Tumor dimensions were collected using calipers, two measurements-longitudinal and transverse were taken for each tumor. These values were multiplied together to give an indication of tumor size. A kill size of 25 mm was set such that any tumor reaching size in one of the two measurable dimension was culled by exposure to a rising concentration of CO₂ and cervical dislocation. Kill curves were then generated.

### Results

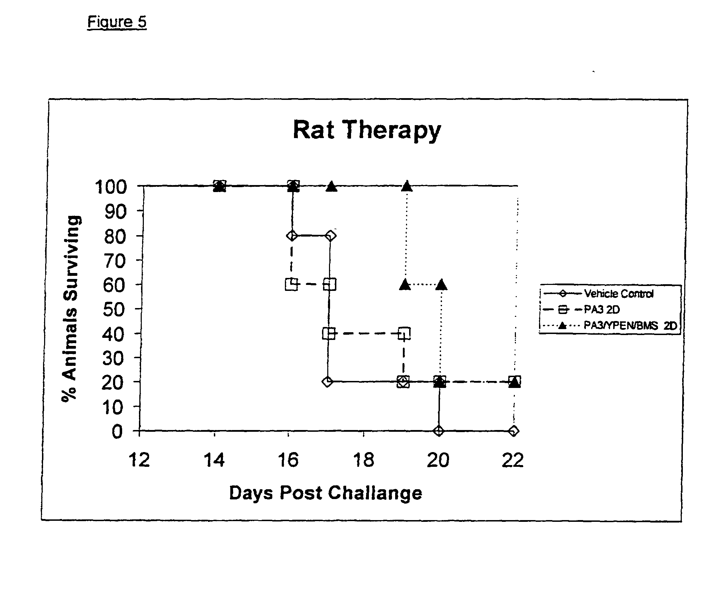

Results for the comparison of 2D grown co-cultures in the rat therapy model, which is an extremely aggressive model with which to work, is shown in Figure 5. The co-culture of PA3 cells with YPEN and RBMS cells resulted in a more efficacious vaccine than PA3 cells alone. It is important to understand that in this experiment the vaccine was administered on a total cell number basis which means that the actual quantity of PA3 cells in the co-culture is significantly lower than the PA3 cells alone supporting the hypothesis that the co-culture results in a more relevant antigen repertoire than PA3 cell alone.

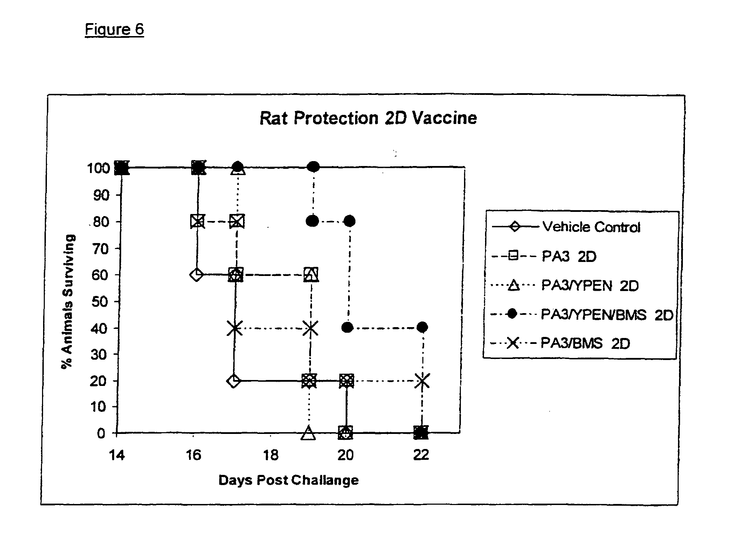

In the rat protection model the results are equally as impressive Figure 6. Firstly the 2D grown PA3 cells offer a small level of protection. The co-cultures of PA3 with YPEN and PA3 with RBMS also offer a low level of protection, but the number of PA3 cells in the co-cultures will be reduced since the vaccine is administered on a total cell number basis. This is particulary true for the PA3/YPEN co-culture the PA3 component will be much reduced since the YPEN grow even more aggressively than do the PA3 cells themselves. The co-culture comprising PA3, YPEN and RBMS shows a significant enhancement of vaccine efficacy manifest by a clear increase in survival benefit in the rats vaccinated with this co-cultured vaccine.

### 3D Co-Culture of Prostate Tumour Line with Bone Stromal Cells and Endothelial Cells Enhances Vaccine Efficacy

The rat PA3 prostate tumour line is a relatively weak allogeneic immunogen in the copenhagen rat challenged with autologous MatLyLu prostate cells. We compared PA3 rat prostate tumour cells grown in 3D microgravity culture and PA3 cells co-cultured with bone stromal fibroblasts and YPEN endothelial cells in 3D microgravity culture as a vaccine in rat therapy and protection models.

### Growth of PA3 Cells in 3D Culture

The roller cassette were filled with 35 ml culture medium (RPMI 1640 10% FCS/2mM Glutamine) through the screw cap, to this 6 x 10⁶ cells suspended 6 ml of culture medium was added. The roller was then topped up to full (approx. 50ml) with -10 ml culture medium and the screw cap replaced. A 20ml syringe fitted with a 20µm filter attached was screwed onto the outlet valve of the roller cassette. A second 20ml syringe fitted with a 20µm was filled with culture medium and screwed onto the inlet valve of the roller cassette. Both valves on the cassette were opened and pressure applied to the inlet syringe to force any remaining air bubbles from within the cassette. Once all the air bubbles had been removed both valves were closed and any remaining culture medium was aspirated off and the valves sealed with stoppers. The roller cassettes were attached to the roller apparatus and rotated at 16 rpm at 37°C/5% CO₂. Cultures were allowed to continue for 3-4 days when they were harvested by unscrewing the cap on the roller and pouring the contents into a 50ml tube. The cells were washed three times by centrifugation and resuspension and stored on ice, prior to irradiation.

### Growth of Co-Cultures in 3D

The same growth methodology was used to produce co-cultures of PA3/YPEN, PA3/RBMS and PA3/YPEN/RBMS in 3D microgravity. The total seed into the 3D Cassette was retained at 6x10⁶ cells (3x10⁶ cells/cell type for two cell types or 2x10⁶ cells /cell type for three cell types). The growth and harvest of the co-cultures is as described in the previous section.

### Irradiation/Cell count.

Tubes containing cell suspensions were irradiated with 6 x 25 Gy (Total: 150 Gy). A cell count is then performed on each sample using a haemocytometer. Briefly 10µl of each cell suspension is mixed with 90µl of tryphan blue and approx. 10µl was loaded under the coverslip of the haemocytometer. A cell count was performed using a x20 objective lens. Each cell suspension was then centrifuged at 18,000 rpm for 3 minutes. The resultant pellet was re-suspended into vaccine freezing medium (RPMI 1640/8%FCS/8%DMSO) at 5 x 10⁶ cells per ml of freezing medium. Suspensions were then aliquoted into cryovials at 1.2ml per vial; vials were then placed into a -80°C freezer overnight prior to storage in liquid nitrogen.

### Injection Protocol (vaccine)

The appropriate vials were removed from storage in liquid nitrogen, thawed and 0.2 ml from the vial was injected into shaved right hind flank of a copenhagen rat. 1 vial is sufficient to inject 5 animals (1 experimental group). See below for injection regime for protection and therapy

### Culture of 'Challenge' Tumor Cell Line

The challenge material used for these experiments is the cell line Mat-Ly-Lu (wild type). Mat-Ly-Lu cells(1 x 10⁶) were seeded into a T175 tissue culture flask in 35 ml of RPMI 1640 culture medium supplemented with 10% FCS/2 mM Glutamine. Cells were allowed to grow until confluent under conditions of 37°C/5% CO₂.

Confluent cells were harvested by washing the monolayer with 10ml Hank's buffered salt solution, followed by 5ml trypsin/EDTA solution to remove cells from the flask. Once all cells had lifted away from the flask surface the trypsin reaction was stopped by the addition of 5ml FCS. The 10ml of cell suspension was collected in a 50ml tube, centrifuged at 18,000 rpm for 3 minutes. The resultant pellet was re-suspended into 10ml PBS and stored on ice. A cell count was performed as detailed above and cell suspension adjusted to 5 x 10⁵ per ml and aliquoted into cryovials at 1.2 ml per vial. Vials were then held on ice prior to injection.

### Injection Protocol (challenge)

Mat-Ly-Lu cells prepared as in the previous section (0.2 ml) were injected into shaved left hind flank of a copenhagen rat. 1 vial is sufficient to inject 5 animals (1 experimental group). See below for injection regime for protection and therapy

### Therapy Model

| | **Day 0** **Challenge** | **Day 2** **Therapy** | **Day 7** **Therapy** | **Day 10** **Therapy** |
|---|---|---|---|---|
| Group 1 | WT MLL 1 x 10⁵ per Rat | Freezing Medium | Freezing Medium | Freezing Medium |
| Group 2 | WT MLL 1 x 10⁵ per Rat | PA3 Clone 1 3D 1 x 10⁶ per Rat | PA3 Clone 1 3D 1 x 10⁶ per Rat | PA3 Clone 1 3D 1 x 10⁶ per Rat |
| Group 3 | WT MLL 1 x 10⁵ per Rat | PA3 Clone 1 + YPEN 3D 1 x 10⁶ per Rat | PA3 Clone 1 + YPEN 3D 1 x 10⁶ per Rat | PA3 Clone 1 + YPEN 3D 1 x 14⁶ per Rat |
| Group 4 | WT MLL 1 x 10⁵ per Rat | PA3 Clone 1 + YPEN + RBMS 3D 1 x 10⁶ per Rat | PA3 Clone 1 + YPEN + RBMS 3D 1 x 10⁶ per Rat | PA3 Clone 1 + YPEN + RBMS 3D 1 x 10⁶ per Rat |
| Group 5 | WT MLL 1 x 10⁵ per Rat | PA3 Clone 1 + RBMS 3D 1 x 10⁶ per Rat | PA3 Clone 1 + RBMS 3D 1 x 10⁶ per Rat | PA3 Clone 1 + RBMS 3D 1 x 10⁶ per Rat |

### Protection Model

| | **Day -14** **Protection** | **Day -7** **Protection** | **Day 0** **Challenge** |
|---|---|---|---|
| Group 1 | Freezing Medium | Freezing Medium | WT MLL 1 x 10⁵ per Rat |
| Group 2 | PA3 Clone 1 3D 1 x 10⁶ per Rat | PA3 Clone 1 3D 1 x 10⁶ per Rat | WT MLL 1 x 10⁵ per Rat |
| Group 3 | PA3 Clone 1 + YPEN 3D 1 x 10⁶ per Rat | PA3 Clone 1 + YPEN 3D 1 x 10⁶ per Rat | WT MLL 1 x 10⁵ per Rat |
| Group 4 | PA3 Clone 1 + YPEN + RBMS 3D 1 x 10⁶ per Rat | PA3 Clone 1 + YPEN + RBMS 3D 1 x 10⁶ per Rat | WT MLL 1 x 10⁵ per Rat |
| Group 5 | PA3 Clone 1 + RBMS 3D 1 x 10⁶ per Rat | PA3 Clone 1 + RBMS 3D 1 x 10⁶ per Rat | WT MLL 1 x 10⁵ per Rat |

### Animal Measurement Criteria

Tumor dimensions were collected using calipers, two measurements-longitudinal and transverse were taken for each tumor. These values were multiplied together to give an indication of tumor size. A kill size of 25 mm was set such that any tumor reaching this size in one of the two measurable dimension was culled by exposure to a rising concentration of CO2 and cervical dislocation. Kill curves were then generated.

### Results

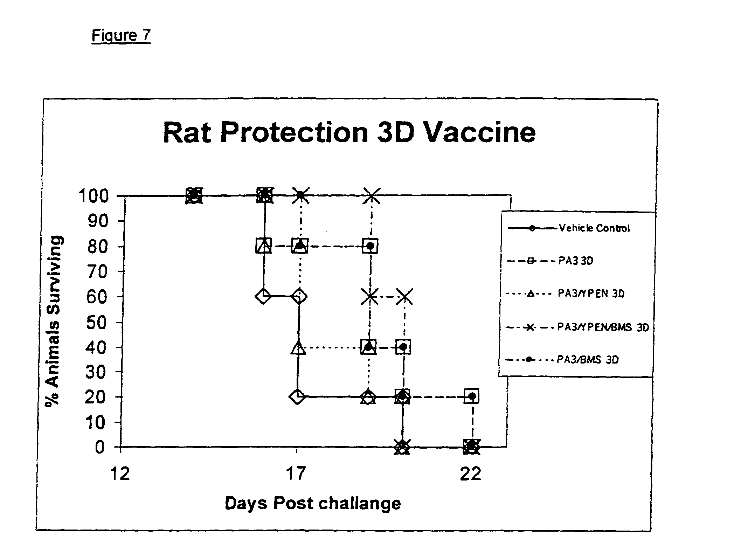

Results for the comparison of 3D grown vaccines is shown in Figure 7. The 3D grown PA3 cells offer a good level of protection above that of the vehicle control. The 3D co-culture of PA3 with YPEN does not confer any protection and may reflect the reduction of PA3 cells as a mention previously . PA3 cells with RBMS also offer a good level of protection even though the level of PA3 is reduced compared to the PA3 alone vaccine. The co-culture of PA3/YPEN and RBMS provides slightly better protection than does the PA3 vaccine even though the PA3 contribution is probably three fold lower due to the seeeding level in the 3D culture. This would suggest that the co-culture of cell has resulted in an improved repertoire of antigens more reflective of the tumour in situ even though the vaccine is allogeneic.

**Table 1**

| **PCR Analysis of prostate cells co-cultured with bone marrow stromal cells** | | | |
|---|---|---|---|
| Antigen | NHBMSC | ONYCAP - 23 | ONYCAP-23 + NHBMSC |
| PSA | - | - | + |
| Androgen receptor | - | - | + |
| MMP-9 | - | - | + |
| Hyaluronic acid receptor | - | - | + |

**Table 2**

| **Genes Down-Regulated in DU-145 / NHPSF Co-Culture** | | | |
|---|---|---|---|
| **Spot** | **Intensity** | | **Gene** |
| Co-Culture | Du-145 | NHPS F | |
| 1 | 6 | 0 | active breakpoint cluster region-related protein |
| 5 | 10 | 0 | aurora- & IPL1-like midbody-associated protein kinase 1 (AIM1); ARK2 |
| 4 | 7 | 0 | cytohesin-1; Sec7p-like protein |
| 9 | 0 | 41 | insulin-like growth factor binding protein 5 precursor |
| 11 | 2 | 23 | placental calcium-binding protein; calvasculin; |
| 18 | 9 | 38 | low-density lipoprotein receptor-related protein 1 precursor (LRP); CD91 antigen |
| 9 | 15 | 1 | glutathione synthetase (GSH synthetase; GSH-S); glutathione synthase |
| 19 | 32 | 1 | paxillin |
| 9 | 14 | 3 | histone deaceytlase 3C |
| 4 | 6 | 0 | CD9 antigen; p24; leukocyte antigen MIC3; motility-related protein (MRP-1) |
| 15 | 42 | 2 | hepatoma-derived growth factor (HDGF) |
| 7 | 1 | 55 | procollagen 1 alpha 2 subunit precursor (COL1A2) |
| 10 | 18 | 3 | macrophage migration inhibitory factor (MIF); glycosylation-inhibiting factor (GIF) |
| 16 | 33 | 0 | bikunin; hepatocyte growth factor activator inhibitor 2 |
| 15 | 29 | 5 | ubiquitin-conjugating enzyme E2 32-kDa complementing protein |
| 3 | 7 | 0 | jagged2 (JAG2) |
| 14 | 31 | 5 | basigin precursor (BSG); leukocyte activation antigen M6; |
| 61 | 8 | 143 | metalloproteinase inhibitor 1 precursor (TIMP1); |
| 12 | 4 | 30 | collagen 6 alpha 1 subunit (COL6A1) |
| 4 | 7 | 0 | fibroblast growth factor 8 (FGF8); androgen-induced growth factor precursor (AIGF) |
| 47 | 77 | 23 | 60S ribosomal protein L10; tumor suppressor QM; laminin receptor homolog |
| 7 | 3 | 3 | P37NB |
| 14 | 63 | 0 | type II cytoskeletal 7 keratin (KRT7); cytokeratin 7 (K7; CK7) |
| 106 | 174 | 28 | 40S ribosomal protein S5 |
| 7 | 0 | 21 | fibronectin precursor (FN) |
| 4 | 8 | 0 | thymidylate synthase (TYMS; TS) |
| 11 | 20 | 0 | type II cytoskeletal 8 keratin (KRT8); cytokeratin 8 (K8; CK8) |
| 4 | 8 | 0 | arginine/serine-rich splicing factor 7; splicing factor 9G8 |
| 5 | 8 | 0 | suppressor for yeast mutant |
| 15 | 0 | 41 | secreted protein acidic and rich in cysteine precursor (SPARC); osteonectin (ON); |
| 95 | 168 | 37 | brain-specific tubulin alpha 1 subunit (TUBA1) |

**Table 3**

| **Genes Up-Regulated in DU-145 / NHPSF Co-Culture** | | | |
|---|---|---|---|
| **Spot Intensity** | | | |
| Co-Culture | DU-145 | Stroma **I** | **Gene** |
| 6 | 2 | 2 | interferon-inducible protein 9-27 |
| 21 | 7 | 12 | cyclin-dependent kinase inh 1 (CDKN1A); (MDA6)(CIP1); WAF1 |
| 28 | 19 | 0 | 14-3-3 protein sigma; stratifin; epithelial cell marker protein 1 |
| 18 | 11 | 4 | DNA excision repair protein ERCC1 |
| 6 | 4 | 3 | BRCA1-associated ring domain protein |
| 6 | 2 | 3 | retinoic acid receptor gamma 1 (RAR-gamma 1; RARG) |
| 6 | 3 | 2 | interferon regulatory factor 3 (IRF3) |
| 44 | 22 | 24 | poly(ADP-ribose) polymerase PARP;poly(ADP-ribose) synthetase |
| 23 | 9 | 10 | early growth response protein 1 (hEGR1); transcrip factor ETR103; |
| 7 | 3 | 1 | leukocyte interferon-inducible peptide |
| 6 | 3 | 0 | HEM45 |
| 7 | 4 | 1 | KIAA0022 GENE |
| 18 | 9 | 0 | uridine phosphorylase (UDRPase) |
| 40 | 24 | 1 | type I cytoskeletal 19 keratin; cytokeratin 19 (K19; CK19) |
| 4 | 6 | 0 | ribosomal protein S21 (RPS21) |
| 40 | 18 | 1 | IGFBP complex acid labile chain |

**Table 4**

| **Genes Down-Regulated in DU-145 / NHBSF Co-Culture** | | | |
|---|---|---|---|
| Spot Intensity and Ratio | | | |
| DU-145/NHBSF | Co-culture | Ratio | |
| 6368 | 1 | 0.000157 | lymphocyte activation gene-3 protein precursor (LAG-3); FDC protein |
| 7296 | 1 | 0.000137 | Syndecan2 (Fibroglycan) (Heparine SULPHATE PROTEOGLYCAN CORE PROTEIN |
| 6988 | 392 | 0.056096 | LGALS3, MAC2 (Galectin-3, MAC-2 antigen, |
| 6088 | 140 | 0.022996 | intercellular adhesion molecule 3 precursor (ICAM3); CDW50 antigen; ICAM-R |
| 8820 | 1 | 0.000113 | endoglin precursor (ENG; END); CD105 antigen |
| 7836 | 1912 | 0.244002 | lymphocyte function-associated antigen 3 precursor (LFA3); CD58 antigen |
| 10096 | 1 | 9.9E-05 | thy-1 membrane glycoprotein precursor; CDW90 antigen |
| 7164 | 28 | 0.003908 | Runt domain-containing protein PEBP2aC1 |
| 9252 | 1948 | 0.210549 | ZINC FINGER PROTEIN 151 (MIZ-1 PROTEIN) |
| 8116 | 2440 | 0.300641 | MADER NUCLEAR PROTEIN |
| 9768 | 3932 | 0.402539 | aldehyde dehydrogenase (ALDH6) |
| 18908 | 11180 | 0.591284 | cytochrome P450 51 (CYP51) + CYP51P1 +CYP51P2 |
| 23200 | 14948 | 0.64431 | annexin IV (ANX4); lipocortin IV; endonexin I; |
| 23700 | 15264 | 0.644051 | annexin VII (ANX7); synexin |
| 13052 | 3736 | 0.28624 | N-ethylmaleimide-sensitive fusion protein (NEM-sensitive fusion protein; NSF) |
| 13724 | 5904 | 0.430195 | vesicle-membrane fusion protein SNAP23A |
| 27152 | 11140 | 0.410283 | protein SEC23 homolog isoform A (SEC23A) |
| 11240 | 5100 | 0.453737 | PROTEIN TRANSPORT PROTEIN SEC23 HOMOLOG ISOFORM B. |
| 10608 | 3868 | 0.36463 | syntaxin 7 (STX7) |
| 12068 | 6016 | 0.498508 | ras-related protein RAB-32 |
| 5600 | 112 | 0.02 | alcohol dehydrogenase class II pi subunit |
| 5780 | 644 | 0.111419 | peroxisomal bifunctonal enzyme |
| 18800 | 10588 | 0.563191 | 3-hydroxy-3-methylglutaryl-coenzyme A reductase (HMG-CoA reductase; HMGCR) |
| 6548 | 828 | 0.126451 | apolipoprotein D precursor (apo-D) |
| 13652 | 8300 | 0.60797 | dihydrofolate reductase (DHFR) |
| 15652 | 10956 | 0.699974 | homolog of yeast D2-isopentenylpyrophosphate isomerase (IPP isomerase) |
| 10036 | 1888 | 0.188123 | LOX (Protein-lysine 6-oxidase, Lysyl-Oxidase) |
| 15136 | 10044 | 0.663584 | TIA-1 related protein; nucleolysin TIAR |
| 5648 | 480 | 0.084986 | NEUROPEPTIDE Y RECEPTOR TYPE 5 (NPY5-R) (Y5 RECEPTOR) (NPYY5) |
| 16000 | 8672 | 0.542 | neuron-derived orphan receptor 1 (NOR1); |
| 17960 | 916 | 0.051002 | Proenkephalin A precursor |
| 14484 | 6760 | 0.466722 | insulin-like growth factor-binding protein 10 (IGFBP10) |
| 12248 | 1 | 8.16E-05 | osteoprotegerin (OPG) |
| 13264 | 7580 | 0.571472 | casein kinase II alpha' subunit (CK II); CSNK2A2 |
| 13184 | 6008 | 0.455704 | serine/threonine protein phosphatase PP1-gamma 1 catalytic subunit (PP-1G) |
| 26140 | 16248 | 0.621576 | ras-related protein RAP-1B; GTP-binding protein SMG p21 B |
| 6264 | 700 | 0.11175 | inositol polyphosphate 1 phosphatase (INPP1; IPP) |
| 15552 | 5680 | 0.365226 | calcium-binding protein ERC-55 precursor |
| 17196 | 10024 | 0.582926 | 14-3-3 protein tau; 14-3-3 protein theta; 14-3-3 protein T-cell; HS1 protein |
| 14720 | 8624 | 0.58587 | amyloid-like protein 2 |
| 11156 | 4312 | 0.386518 | MYOSIN PHOSPHATASE TARGET SUBUNIT 1. |
| 5956 | 880 | 0.14775 | microtubule-associated protein tau (MAPT); MTBT1 |
| 7540 | 1100 | 0.145889 | PROTEIN PHPS1-2 |
| 7428 | 916 | 0.123317 | epilepsy holoprosencephaly candidate-1 protein (EHOC-1); transmembrane protein 1 |
| 13184 | 6080 | 0.461165 | DNA polymerase gamma (POLG); mitochondrial DNA polymerase |

**Table 5**

| **Genes Up-Regulated in DU-145 / NHBSF Co-Culture** | | | |
|---|---|---|---|
| Spot Intensity and Ratio | | | |
| DU-145/NHBSF | Co-culture | Ratio | |
| 3768 | 10280 | 2.728238 | ESE1, ERT, JEN, ELF3 (Epithelial-specific transcription factor) |
| 3732 | 13808 | 3.699893 | Myelin-oligodendrocyte glycoprotein precursor (MOG) |
| 19928 | 32924 | 1.652148 | HLA-B-associated transcript 2; large proline-rich protein BAT2 |
| 2388 | 9900 | 4.145729 | Complement 3 (C3) |
| 6552 | 14756 | 2.252137 | breakpoint cluster region protein (BCR) |
| 908 | 9648 | 10.62555 | erythroblastosis virus oncogene homolog 1 (ETS-1); p54 |
| 5072 | 12840 | 2.531546 | zinc finger protein hrx; ALL-1; MLL |
| 3400 | 10992 | 3.232941 | ENL protein |
| 3260 | 9544 | 2.927607 | ZINC FINGER PROTEIN RFP (RET FINGER PROTEIN) |
| 5360 | 9420 | 1.757463 | nuclear pore complex protein 214 (NUP214); nucleoporin |
| 9300 | 24168 | 2.59871 | retinoic acid receptor alpha |
| 2416 | 11840 | 4.900662 | major vault protein (MVP); lung resistance-related protein (LRP) |
| 5472 | 36892 | 6.741959 | cartilage glycoprotein 39 precursor (GP39); |
| 4728 | 10836 | 2.291878 | ras-related protein RAB-31 |
| 8204 | 12364 | 1.50707 | RAB GDP dissociation inihibitor alpha |
| 6600 | 17404 | 2.63697 | calcium-binding protein p22; calcium-binding protein CHP |
| 7328 | 14264 | 1.946507 | hexokinase 1 (HK 1); brain-form hexokinase |
| 4768 | 12128 | 2.543624 | CREATINE KINASE B CHAIN |
| 13484 | 21100 | 1.564818 | phosphatidylethanolamine-binding protein; neuropolypeptide |
| 556 | 13308 | 23.93525 | cystathionine beta-synthase |
| 10436 | 18632 | 1.785358 | GLCLC, GLCL (gamma-glutamylcysteine synthetase) |
| 2904 | 10360 | 3.567493 | eosinophil peroxidase precursor (EPO; EPP); EPER |
| 6912 | 10828 | 1.566551 | FRATAXIN = FRDA FRIEDREICH'S ATAXIA PROTEIN |
| 2988 | 12356 | 4.135207 | lysosomal acid phosphatase precursor (LAP); ACP2 |
| 1540 | 12788 | 8.303896 | platelet-activating factor acetylhydrolase IB gamma subunit |
| 4612 | 13784 | 2.988725 | cyclophilin 3 protein (CYP3); |
| 1432 | 12328 | 8.608939 | SCA2 |
| 3548 | 14756 | 4.158963 | leukocyte platelet-activating factor receptor |
| 3760 | 9524 | 2.532979 | casein kinase II alpha subunit (CK II); CSNK2A1 |
| 10172 | 22768 | 2.238301 | cathepsin A; carboxypeptidase C; PPGB |
| 10940 | 24016 | 2.195247 | cathepsin B precursor (CTSB); preprocathepsin B; APP secretase |
| 6992 | 15832 | 2.264302 | calpain 1 large (catalytic) subunit; |
| 5844 | 10152 | 1.737166 | lipoprotein-associated coagulation inhibitor |

## Claims

1. A whole cell cancer vaccine having:
an immunogenic component which comprises co-cultured malignant and non-malignant cells; and
a pharmaceutically acceptable carrier, excipient on diluent.

2. A whole cell vaccine according to claim 1 wherein the co-cultured cells have an altered immunogenicity.

3. A whole cell vaccine according to claim 2 wherein the co-cultured cells were cultured under conditions which were not conventional static 2D culture conditions and wherein the immunogenicity of the co-cultured cells was altered in comparison with the immunogenicity of equivalent cells co-cultured under conventional static 2D culture conditions.

4. A whole cell cancer vaccine according to claim 2 or 3 wherein the altered immunogenicity is caused by presentation of peptides on MHC-1 molecules of the non-malignant cells.

5. A whole cell cancer vaccine according to claim 1 wherein the co-cultured cells are in the form of a spheroid.

6. A whole cell vaccine according to claim 5 wherein one or more of the co-cultured cells of the spheroid has an altered immunogenicity.

7. A whole cell vaccine according to claim 6 wherein the co-cultured cells were co-cultured under microgravity and the or each co-cultured cell has an altered immunogenicity compared to equivalent cells co-cultured under conventional static 2D culture conditions.

8. A whole cell cancer vaccine according to claim 6 or 7 wherein the altered immunogenicity is caused by presentation of peptides on MHC-1 molecules of the non-malignant cells.

9. A whole cell cancer vaccine according to any preceding claim wherein the immunogenic component further comprises an adjuvant.

10. A whole cell vaccine according to any preceding claim wherein the non-malignant cells are autologous and the malignant cells are allogeneic.

11. A whole cell cancer vaccine according to any preceding claim wherein the malignant and/or non-malignant cells are derived from a cell line.

12. A method for producing a whole cell cancer vaccine which comprises:
co-culturing malignant and non-malignant cells; harvesting the co-cultured cells;
adding the harvested co-cultured cells to a pharmaceutically acceptable carrier, excipient or diluent.

13. A method according to claim 12 wherein the cells are co-cultured under conditions which are not conventional static 2D culture conditions.

14. A method according to claim 13 wherein the cells are co-cultured under non-adherent culture conditions.

15. A method according to claim 14 wherein the cells are co-cultured in non adherent culture vessels.

16. A method according to claim 13 wherein the cells are co-cultured under adherent non-static culture conditions.

17. A method according to claim 16 wherein the cells are co-cultured in roller culture bottles.

18. A method according to claim 13 wherein the cells are co-cultured under under microgravity.

19. A method according to claim 18 wherein the cells form spheroids under microgravity and the spheroids are harvested and added to the pharmaceutically acceptable carrier, excipient or diluent.

20. A method according to claim 13 wherein the cells are co-cultured on microcarriers suspended in culture medium.

21. A method according to claim 20 wherein the microcarriers are beads or non woven fibre discs.

22. A method according to any of claims 12 to 21 wherein co-culturing the cells alters their immunogenicity.

23. A method according to claim 22 wherein the immunogenicity of the cells is altered in comparison with equivalent cells co-cultured under conventional static 2D conditions.

24. A method according to claim 22 or 23 wherein the altered immunogenicity is caused by presentation of peptides on MHC-1 molecules of the non-malignant cells.

25. A method according to any of claims 12 to 24 wherein the non-malignant cells are autologous and the malignant cells are allogeneic.

26. A method according to any of claims 12 to 25 wherein the malignant and/or non-malignant cells are derived from a cell line.

27. A method according to any of claims 12 to 26 further comprising gamma irradiating the co-cultured cells.

28. A method according to any of claims 12 to' 27 wherein the cells include one or both of ECACC 00032802 and ECACC 00032801.

29. A vaccine according to any of claims 1 to 11 wherein the cells include one or both of ECACC 00032802 and ECACC 00032801.

30. A vaccine according to any of claims 1 to 11 and 29 wherein the cancer to be treated is prostate cancer.

## Patentansprüche

1. Whole-Cell-Krebsimpfstoff, umfassend:
eine immunogene Komponente, die co-kultivierte maligne und nichtmaligne Zellen umfasst; und
einen pharmazeutisch annehmbaren Träger, ein solches Bindemittel oder Verdünnungsmittel.

2. Whole-Cell-Impfstoff nach Anspruch 1, wobei die co-kultivierten Zellen eine veränderte Immunogenität haben.

3. Whole-Cell-Impfstoff nach Anspruch 2, wobei die co-kultivierten Zellen unter Bedingungen kultiviert wurden, die keine konventionellen statischen 2D-Kulturbedingungen waren, und wobei die Immunogenität der co-kultivierten Zellen im Vergleich zur Immunogenität äquivalenter Zellen, die unter konventionellen statischen 2D-Kulturbedingungen co-kultiviert wurden, verändert wurde.

4. Whole-Cell-Krebsimpfstoff nach Anspruch 2 oder 3, wobei die veränderte Immunogenität durch eine Präsentation von Peptiden auf MHC-1 Molekülen der nichtmalignen Zellen verursacht wird.

5. Whole-Cell-Krebsimpfstoff nach Anspruch 1, wobei die co-kultivierten Zellen die Form eines Sphäroids haben.

6. Whole-Cell-Impfstoff nach Anspruch 5, wobei eine oder mehrere der co-kultivierten Zellen des Sphäroids eine veränderte Immunogenität hat/haben.

7. Whole-Cell-Impfstoff nach Anspruch 6, wobei die co-kultivierten Zellen unter Mikroschwerkraft co-kultiviert wurden und die oder jede co-kultivierte Zelle eine veränderte Immunogenität im Vergleich zu äquivalenten Zellen hat, die unter konventionellen statischen 2D-Kulturbedingungen co-kultiviert wurden.

8. Whole-Cell-Krebsimpfstoff nach Anspruch 6 oder 7, wobei die veränderte Immunogenität durch die Präsentation von Peptiden auf MHC-1 Molekülen der nichtmalignen Zellen verursacht wird.

9. Whole-Cell-Krebsimpfstoff nach einem der vorherigen Ansprüche, wobei die immunogene Komponente ferner ein Adjuvans umfasst.

10. Whole-Cell-Impfstoff nach einem der vorherigen Ansprüche, wobei die nichtmalignen Zellen autolog und die malignen Zellen allogen sind.

11. Whole-Cell-Krebsimpfstoff nach einem der vorherigen Ansprüche, wobei die malignen und/oder nichtmalignen Zellen von einer Zelllinie stammen.

12. Verfahren zur Herstellung eines Whole-Cell-Krebsimpfstoffs, umfassend:
das Co-Kultivieren maligner und nichtmaligner Zellen; Ernten der co-kultivierten Zellen;
Zugeben der geernteten co-kultivierten Zellen zu einem pharmazeutisch annehmbaren Träger, Bindemittel oder Verdünnungsmittel.

13. Verfahren nach Anspruch 12, wobei die Zellen unter Bedingungen co-kultiviert werden, die keine konventionellen statischen 2D-Kulturbedingungen sind.

14. Verfahren nach Anspruch 13, wobei die Zellen unter nichtadhärenten Kulturbedingungen co-kultiviert werden.

15. Verfahren nach Anspruch 14, wobei die Zellen in nichtadhärenten Kulturgefäßen co-kultiviert werden.

16. Verfahren nach Anspruch 13, wobei die Zellen unter adhärenten nichtstatischen Kulturbedingungen co-kultiviert werden.

17. Verfahren nach Anspruch 16, wobei die Zellen in Rollerflaschen co-kultiviert werden.

18. Verfahren nach Anspruch 13, wobei die Zellen unter Mikroschwerkraft co-kultiviert werden.

19. Verfahren nach Anspruch 18, wobei die Zellen Sphäroide unter Mikroschwerkraft bilden und die Sphäroide geerntet und zu dem pharmazeutisch annehmbaren Träger, Bindemittel oder Verdünnungsmittel gegeben werden.

20. Verfahren nach Anspruch 13, wobei die Zellen auf in Kulturmedium suspendierten Mikroträgern co-kultiviert werden.

21. Verfahren nach Anspruch 20, wobei die Mikroträger Kügelchen oder Faservliesscheiben sind.

22. Verfahren nach einem der Ansprüche 12 bis 21, wobei durch das Co-Kultivieren der Zellen ihre Immunogenität verändert wird.

23. Verfahren nach Anspruch 22, wobei die Immunogenität der Zellen im Vergleich zu äquivalenten Zellen, die unter konventionellen statischen 2D-Bedingungen co-kultiviert werden, verändert wird.

24. Verfahren nach Anspruch 22 oder 23, wobei die veränderte. Immunogenität durch eine Präsentation von Peptiden auf MHC-1 Molekülen der nichtmalignen Zellen verursacht wird.

25. Verfahren nach einem der Ansprüche 12 bis 24, wobei die nichtmalignen Zellen autolog und die malignen Zellen allogen sind.

26. Verfahren nach einem der Ansprüche 12 bis 25, wobei die malignen und/oder nichtmalignen Zellen von einer Zelllinie stammen.

27. Verfahren nach einem der Ansprüche 12 bis 26, ferner umfassend das Gammabestrahlen der co-kultivierten Zellen.

28. Verfahren nach einem der Ansprüche 12 bis 27, wobei die Zellen ECACC 00032802 und/oder ECACC 00032801 umfassen.

29. Impfstoff nach einem der Ansprüche 1 bis 11, wobei die Zellen ECACC 00032802 und/oder ECACC 00032801 umfassen.

30. Impfstoff nach einem der Ansprüche 1 bis 11 und 29, wobei der zu behandelnde Krebs Prostatakrebs ist.

## Revendications

1. Vaccin contre le cancer à base de cellules complètes ayant :
un composant immunogène qui comprend des cellules malignes et non malignes co-cultivées; et
un véhicule, un excipient ou un diluant pharmaceutiquement acceptables.

2. Vaccin à base de cellules complètes selon la revendication 1, dans lequel les cellules co-cultivées ont une immunogénicité modifiée.

3. Vaccin à base de cellules complètes selon la revendication 2, dans lequel les cellules co-cultivées ont été cultivées dans des conditions qui n'étaient pas des conditions de culture 2D statique conventionnelle et dans lequel l'immunogénicité des cellules co-cultivées a été modifiée en comparaison à l'immunogénicité de cellules équivalentes co-cultivées dans des conditions de culture 2D statique conventionnelle.

4. Vaccin contre le cancer à base de cellules complètes selon la revendication 2 ou 3, dans lequel l'immunogénicité modifiée est causée par la présentation de peptides sur des molécules MHC-1 des cellules non malignes.

5. Vaccin contre le cancer à base de cellules complètes selon la revendication 1, dans lequel les cellules co-cultivées sont sous forme d'un sphéroïde.

6. Vaccin à base de cellules complètes selon la revendication 5, dans lequel une ou plusieurs des cellules co-cultivées du sphéroïde, a/ont une immunogénicité modifiée.

7. Vaccin à base de cellules complètes selon la revendication 6, dans lequel les cellules co-cultivées ont été co-cultivées sous microgravité et la ou chaque cellule co-cultivée a une immunogénicité modifiée comparée à des cellules équivalentes co-cultivées dans des conditions de culture 2D statique conventionnelle.

8. Vaccin contre le cancer à base de cellules complètes selon la revendication 6 ou 7, dans lequel l'immunogénicité modifiée est causée par la présentation de peptides sur des molécules MHC-1 des cellules non malignes.

9. Vaccin contre le cancer à base de cellules complètes selon l'une quelconque des revendications précédentes, dans lequel le composant immunogène comprend en plus un adjuvant.

10. Vaccin à base de cellules complètes selon l'une quelconque des revendications précédentes, dans lequel les cellules non malignes sont autologues et les cellules malignes sont allogéniques.

11. Vaccin contre le cancer à base de cellules complètes selon l'une quelconque des revendications précédentes, dans lequel les cellules malignes et/ou non malignes sont dérivées d'une lignée cellulaire.

12. Procédé pour produire un vaccin contre le cancer à base de cellules complètes qui comprend :
co-cultiver des cellules malignes et non malignes;
récolter les cellules co-cultivées;
ajouter les cellules co-cultivées récoltées à un véhicule, excipient ou diluant pharmaceutiquement acceptables.

13. Procédé selon la revendication 12, dans lequel les cellules sont co-cultivées dans des conditions qui ne sont pas des conditions de culture 2D statique conventionnelle.

14. Procédé selon la revendication 13, dans lequel les cellules sont co-cultivées dans des conditions de culture non adhérentes.

15. Procédé selon la revendication 14, dans lequel les cellules sont co-cultivées dans des récipients de culture non adhérents.

16. Procédé selon la revendication 13, dans lequel les cellules sont co-cultivées dans des conditions de culture non statiques adhérentes.

17. Procédé selon la revendication 16, dans lequel les cellules sont co-cultivées dans des flacons de culture roulants.

18. Procédé selon la revendication 13, dans lequel les cellules sont co-cultivées sous microgravité.

19. Procédé selon la revendication 18, dans lequel les cellules forment des sphéroïdes sous microgravité et les sphéroïdes sont récoltés et ajoutés au véhicule, excipient ou diluant pharmaceutiquement acceptables.

20. Procédé selon la revendication 13, dans lequel les cellules sont co-cultivées sur des micro-supports mis en suspension dans un milieu de culture.

21. Procédé selon la revendication 20, dans lequel les micro-supports sont des perles ou des disques en fibres non tissées.

22. Procédé selon l'une quelconque des revendications 12 à 21, dans lequel co-cultiver les cellules modifie leur immunogénicité.

23. Procédé selon la revendication 22, dans lequel l'immunogénicité des cellules est modifiée en comparaison à des cellules équivalentes co-cultivées dans des conditons de culture 2D statique conventionnelle.

24. Procédé selon la revendication 22 ou 23, dans lequel l'immunogénicité modifiée est causée par la présentation de peptides sur des molécules MHC-1 des cellules non malignes.

25. Procédé selon l'une quelconque des revendications 12 à 24, dans lequel les cellules non malignes sont autologues et les cellules malignes sont allogéniques.

26. Procédé selon l'une quelconque des revendications 12 à 25, dans lequel les cellules malignes et/ou non malignes sont dérivées d'une lignée cellulaire.

27. Procédé selon l'une quelconque des revendications 12 à 26 comprenant en plus l'irradiation gamma des cellules co-cultivées.

28. Procédé selon l'une quelconque des revendications 12 à 27, dans lequel les cellules comprennent l'un ou les deux de ECACC 00032802 et de ECACC 00032801.

29. Vaccin selon l'une quelconque des revendications 1 à 11, dans lequel les cellules comprennent l'un ou les deux de ECACC 00032802 et de ECACC 00032801.

30. Vaccin selon l'une quelconque des revendications 1 à 11 et 29, dans lequel le cancer à traiter est un cancer de la prostate.
